# EUROPEAN PATENT APPLICATION

(11) **EP 4 535 008 A1**
(43) Date of publication of application: **09.04.2025**
(21) Application number: 23815676.4
(22) Date of filing: 02.05.2023
(51) Int. Cl.: G01N 35/02, G01N 35/00

(54) **INSPECTION DEVICE**

(30) Priority: 03.06.2022 JP 2022091058
(71) Applicant: FUJIFILM Corporation, Tokyo 106-8620 (JP)
(72) Inventor: MORI, Takaaki, Ashigarakami-gun, Kanagawa 258-8538 (JP); DENAWA, Tatsuyuki, Ashigarakami-gun, Kanagawa 258-8538 (JP)
(74) Representative: Dehns Germany Partnerschaft mbB
(86) International application number: PCT/JP2023/017167
(87) International publication number: WO 2023/233915

(57) **Abstract**

An examination apparatus includes a measurement line that performs a measurement process while transporting a cartridge for measurement along a transport passage for measurement, a pre-treatment line that performs a pre-treatment while transporting a cartridge for pre-treatment along a transport passage for pre-treatment, and a specimen transfer mechanism that transfers a pre-treated specimen in which a pre-treatment is completed from the cartridge for pre-treatment in the pre-treatment line to the cartridge for measurement in the measurement line. The transport passage for pre-treatment and the transport passage for measurement are arranged such that a pre-treatment end position in the transport passage for pre-treatment, at which the pre-treatment is completed, is closer to an upstream side in a transport direction of the transport passage for measurement than a downstream side in the transport direction.

## Description

### BACKGROUND OF THE INVENTION

### 1. Field of the Invention

The present invention relates to an examination apparatus.

### 2. Description of the Related Art

An examination apparatus which quantitatively or qualitatively detects an examination target substance in a specimen has been known. Such an examination apparatus mainly utilizes an immunoassay principle, and examples thereof include a chemiluminescent enzyme immunological measuring apparatus and a fluorescence immunological analysis apparatus.

In such an examination apparatus, the examination target substance is optically detected by detecting luminescence or fluorescence based on a label such as an enzyme label and a fluorescent label, which has been imparted to the examination target substance in the specimen by an immunoreaction. An examination apparatus in which a process of imparting the label to such a target substance in a specimen and an optically detecting process of the examination target substance are automated has been proposed.

As the automated examination apparatus, a configuration in which a plurality of reaction containers are arranged on a rotary disk, and respective processing sections such as specimen dispensing, reagent dispensing, and light detection are assigned to predetermined rotational angular positions, and an examination is performed while the rotary disk is rotated once has been known (JP2009-31204A).

In addition, as the automated examination apparatus, an examination apparatus which uses a cartridge including a cell in which a reagent used in one examination is accommodated and a reaction cell in which a specimen is dispensed and which linearly transports the cartridge has also been known (JP1993-40122A (JP-H5-40122A)).

The examination apparatus disclosed in JP1993-40122A (JP-H5-40122A) uses a single-use type cartridge, which is different from the examination apparatus disclosed in JP2009-31204A. In addition, the examination apparatus of JP1993-40122A (JP-H5-40122A) has a linear transport passage as a transport passage for transporting the cartridge, and each processing section related to the measurement process of the examination target substance, such as specimen dispensing, reagent dispensing, and light detection, is disposed on the transport passage, the cartridge is transported along the transport passage, and each process is performed in each processing section.

### SUMMARY OF THE INVENTION

In recent years, in immunoassays, a method of performing pre-treatment such as suppressing activity of a substance which inhibits an immunoreaction in a specimen before the immune reaction has been proposed. Depending on examination item (for example, the examination target substance to be detected), detection accuracy can be improved by performing the pre-treatment. The pre-treatment referred to here is a treatment of heating a specimen in a state of being mixed with a pre-treatment liquid. Such pre-treatment is also performed in the examination apparatus disclosed in JP2009-31204A.

Different from the examination apparatus disclosed in JP2009-31204A, which performs the measurement process while rotating a rotary disk having a reaction container, the examination apparatus disclosed in JP1993-40122A (JP-H5-40122A) has a measurement line for performing the measurement process while linearly transporting a cartridge. In addition, the examination apparatus disclosed in JP1993-40122A (JP-H5-40122A) uses a single-use type cartridge, which is different from the examination apparatus disclosed in JP2009-31204A. In a case where a cartridge is used, the linear measurement line disclosed in JP1993-40122A (JP-H5-40122A) may be advantageous as compared with the rotary disk disclosed in JP2009-31204A in terms of simplification and reduction in size of a transport mechanism for transporting the cartridge and a mechanism for detaching the cartridge from the transport passage.

Therefore, even in an examination apparatus having a measurement line for linearly transporting the cartridge as disclosed in JP1993-40122A (JP-H5-40122A), there has been a demand for enabling the execution of the pre-treatment as in the examination apparatus disclosed in JP2009-31204A.

The examination apparatus disclosed in JP2009-31204A has a rotary disk for pre-treatment, which performs the pre-treatment, in addition to a rotary disk for measurement, which performs the measurement process, and further has a specimen transfer mechanism which transfers a pre-treated specimen from a container disposed on the rotary disk for pre-treatment to the rotary disk for measurement. During the transfer of the specimen, there is a concern about contamination due to dripping of the specimen. In a case where the rotary disk is used as in the examination apparatus disclosed in JP2009-31204A, since the reaction container disposed on the rotary disk periodically returns to the same position, the rotary disk for measurement and the rotary disk for pre-treatment are arranged side by side, and the specimen is transferred at a position where reaction containers as a transfer source and a transfer destination of the specimen are close to each other, a specimen transfer distance can be relatively shortened, and thus there is little concern about risk of contamination due to the specimen transfer.

However, in the case of the examination apparatus which linearly transports the cartridge as disclosed in JP1993-40122A (JP-H5-40122A), the cartridge does not return to the same position periodically, and thus there is a problem that there is a concern about the risk of contamination due to the specimen transfer, as in the case of the examination apparatus disclosed in JP2009-31204A.

An object of the present disclosure is to provide an examination apparatus that linearly transports a cartridge for measurement, which is capable of performing pre-treatment and suppressing occurrence of contamination due to transfer of a pre-treated specimen in the examination apparatus.

An examination apparatus according to the present disclosure is an examination apparatus for performing a measurement process using a cartridge for measurement, which has a reaction cell in which a specimen is dispensed and mixed with a reagent, the examination apparatus including
a measurement line that has a transport passage for measurement, which linearly transports the cartridge for measurement in one direction from an upstream side to a downstream side, and that performs the measurement process while transporting the cartridge for measurement along the transport passage for measurement;
a pre-treatment line that performs a pre-treatment in which, before performing the measurement process, the specimen is heated in a state in which the specimen is mixed with a pre-treatment liquid, the pre-treatment line having a transport passage for pre-treatment, which transports a cartridge for pre-treatment used in the pre-treatment, the cartridge for pre-treatment being different from the cartridge for measurement, and the pre-treatment line performing the pre-treatment while transporting the cartridge for pre-treatment along the transport passage for pre-treatment; and
a specimen transfer mechanism that transfers a pre-treated specimen in which the pre-treatment is completed, from the cartridge for pre-treatment in the pre-treatment line to the cartridge for measurement in the measurement line,
in which the transport passage for pre-treatment and the transport passage for measurement are arranged such that a pre-treatment end position in the transport passage for pre-treatment, at which the pre-treatment is completed, is closer to the upstream side in a transport direction of the transport passage for measurement than the downstream side in the transport direction.

The transport passage for pre-treatment may be a transport passage that linearly transports the cartridge for pre-treatment in one direction from an upstream side to a downstream side, similarly to the transport passage for measurement.

It is preferable that the transport passage for pre-treatment and the transport passage for measurement are arranged such that the downstream side in the transport direction of the transport passage for pre-treatment is closest to the upstream side in the transport direction of the transport passage for measurement.

The transport passage for pre-treatment and the transport passage for measurement may be arranged in series so as to be connected in a straight line.

The transport passage for pre-treatment and the transport passage for measurement may be arranged such that the transport directions are parallel to each other and the downstream side of the transport passage for pre-treatment and the upstream side of the transport passage for measurement partially overlap each other.

The transport passage for pre-treatment and the transport passage for measurement may be arranged such that the transport directions are parallel to each other and the upstream side and the downstream side of each transport direction are opposite to each other.

The transport passage for pre-treatment and the transport passage for measurement may be arranged such that the transport directions are orthogonal to each other.

At least one of the transport passage for pre-treatment or the transport passage for measurement may be provided a plurality.

The transport passage for pre-treatment may have an annular shape.

It is preferable that, in the transport passage for pre-treatment, at least one of a pre-treatment start position at which the pre-treatment is started or the pre-treatment end position is variable.

The specimen transfer mechanism may be a dispensing mechanism that suctions the pre-treated specimen from the cartridge for pre-treatment and discharges the pre-treated specimen to the cartridge for measurement.

It is preferable that the dispensing mechanism is capable of suctioning the pre-treated specimen from the cartridge for pre-treatment at any position on the transport passage for pre-treatment.

It is preferable that the measurement line and the pre-treatment line each have a heating unit which heats the specimen, and each heating unit is controllable to a different temperature.

According to the technology of the present disclosure, it is possible to provide an examination apparatus that linearly transports a cartridge, which is capable of performing pre-treatment and suppressing occurrence of contamination due to transfer of a pre-treated specimen in the examination apparatus.

### BRIEF DESCRIPTION OF THE DRAWINGS

Fig. 1 is a schematic diagram showing an overall configuration of an examination apparatus according to a first embodiment.
Figs. 2A and 2B are schematic views of an example of a cartridge for measurement, in which Fig. 2A is a top view of the cartridge for measurement and Fig. 2B is a front view of the cartridge for measurement.
Fig. 3 is a process diagram showing a procedure of a measurement process based on a chemiluminescent enzyme immunoassay method.
Fig. 4 is a perspective view of a cartridge for pre-treatment and a cartridge for measurement.
Fig. 5 is a perspective view schematically showing a configuration of a pre-treatment line, a measurement line, and a dispensing mechanism.
Fig. 6 is a plan view of a transport mechanism for measurement.
Fig. 7 is a cross-sectional view taken along a line VI-VI in Fig. 6.
Fig. 8 is a view schematically showing a step of moving the cartridge for measurement in a transport direction with movement of a heat block.
Fig. 9 is a plan view showing an arrangement of a transport passage for pre-treatment and a transport passage for measurement in the examination apparatus.
Fig. 10 is a plan view showing an arrangement of a transport passage for pre-treatment and a transport passage for measurement in a modification example.
Fig. 11 is a perspective view of a cartridge moving mechanism.
Fig. 12 is a plan view schematically showing a configuration of a pre-treatment line, a measurement line, and a dispensing mechanism of an examination apparatus according to a second embodiment.
Fig. 13 is a view showing a modification example of the arrangement of the transport passage for pre-treatment and the transport passage for measurement.
Fig. 14 is a view showing a modification example of the arrangement of the transport passage for pre-treatment and the transport passage for measurement.
Fig. 15 is a view showing a modification example of the arrangement of the transport passage for pre-treatment and the transport passage for measurement.
Fig. 16 is a view showing a modification example of the arrangement of the transport passage for pre-treatment and the transport passage for measurement.
Fig. 17 is a view showing a modification example of the arrangement of the transport passage for pre-treatment and the transport passage for measurement.
Fig. 18 is a view showing a modification example of the arrangement of the transport passage for pre-treatment and the transport passage for measurement.
Fig. 19 is a view showing a modification example of the arrangement of the transport passage for pre-treatment and the transport passage for measurement.

### DESCRIPTION OF THE PREFERRED EMBODIMENTS

Hereinafter, an examination apparatus according to the embodiment of the present disclosure will be described with reference to the drawings. Constituent elements indicated by the same reference numeral in the drawings mean the same constituent element. However, unless otherwise specified in the specification, each constituent element is not limited to one and may be plural.

### "Examination apparatus according to first embodiment"

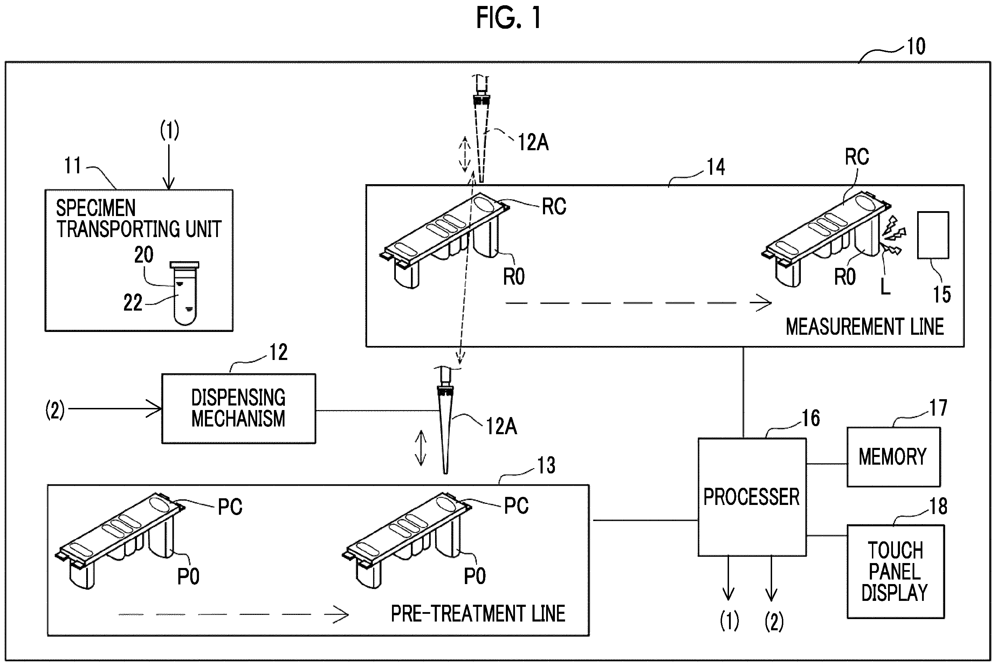

Fig. 1 is a schematic diagram showing an overall configuration of an examination apparatus 10 according to a first embodiment. As an example, the examination apparatus 10 is an immunological analysis apparatus which detects an examination target substance in a specimen collected from a biological body by an antigen-antibody reaction. The examination apparatus 10 optically detects the examination target substance in a specimen using a cartridge RC for measurement, and outputs an examination result.

As an example, the specimen 22 is a body fluid such as blood, collected from the biological body. In a case where the specimen 22 is blood, the specimen 22 may be whole blood, blood plasma, serum, or the like. In addition, a centrifuge may be provided in the examination apparatus 10 to extract blood plasma or serum from whole blood. Furthermore, the examination target substance A which can be contained in the specimen 22 is an antigen, an antibody, a protein, a low-molecular-weight compound, or the like. The specimen 22 is not limited to the blood, and may be a substance collected from the biological body, such as urine and body fluid. A specimen collection container 20 which accommodates the specimen 22 to be examined is loaded into the examination apparatus 10 and is subjected to examination. A plurality of the specimen collection containers 20 may be collectively loaded into the examination apparatus 10 such that the examination apparatus 10 can continuously process examinations for the plurality of the specimens 22.

The cartridge RC for measurement is attachably and detachably loaded into the examination apparatus 10. The cartridge RC for measurement is a single-use type used once for one specimen 22. As an example, the cartridge RC for measurement includes all reagent necessary for the examination of the specimen 22.

As an example, the examination apparatus 10 according to the present example performs an examination based on a chemiluminescent enzyme immunoassay method.

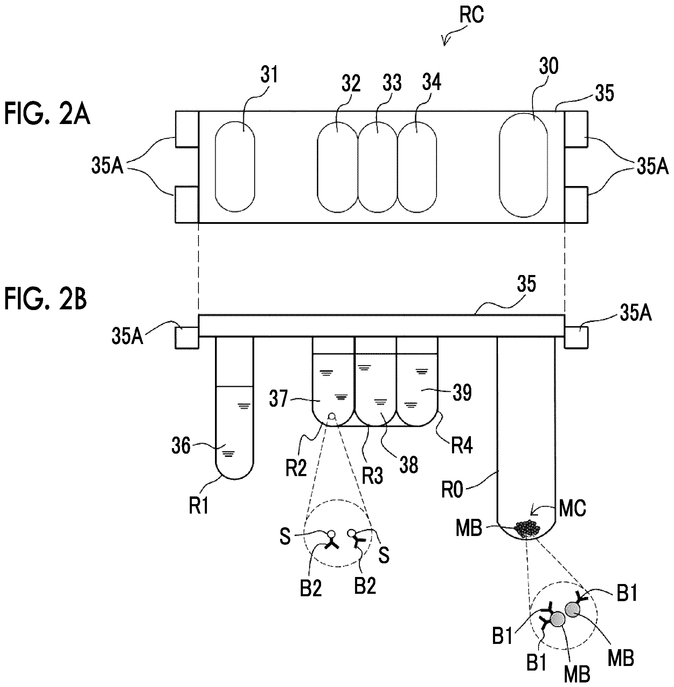

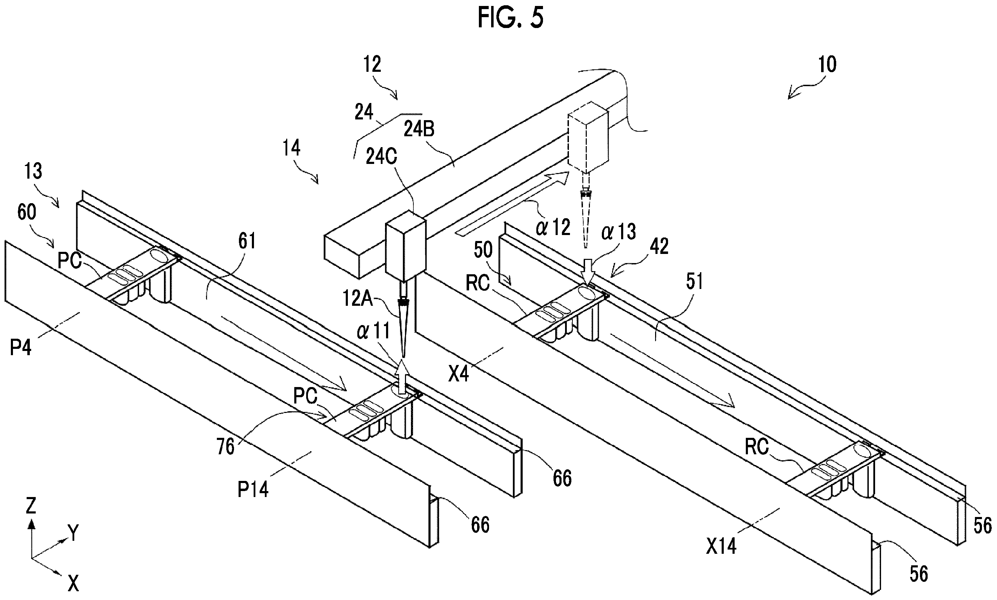

Figs. 2A and 2B are schematic views of an example of the cartridge RC for measurement, in which Fig. 2A is a top view of the cartridge RC for measurement and Fig. 2B is a front view of the cartridge RC for measurement. The cartridge RC for measurement includes a plate-shaped connection portion 35 having five openings 30 to 34, and five tubular cells R0 to R4 each having one end of each of the openings 30 to 34 and extending downward to include the reaction cell R0. Flange portions 35A are provided at both ends of the connection portion 35. The present examination apparatus 10 includes a transport mechanism 50 for measurement, which transports the cartridge RC for measurement, and the cartridge RC for measurement is held by the transport mechanism 50 for measurement and is transported along a transport passage 51 for measurement (see Fig. 5) in the transport mechanism 50 for measurement. The flange portion 35A functions as an engaging portion which engages with the transport mechanism 50 for measurement. The cartridge RC for measurement has a configuration in which the plurality of cells R0 to R4 are integrated by the connection portion 35. Among the plurality of cells R0 to R4, the reaction cell R0 and the cell R1 disposed at both ends are longer than the other cells R2 to R4. The reaction cell R0 is the longest. Before use, the openings 30 to 34 of the cartridge RC for measurement are covered with a sealing film (not shown).

The reaction cell R0 contains a plurality of magnetic particles MB modified with a first binding substance B1 for specifically binding to the examination target substance A. The specimen 22 is dispensed into the reaction cell R0, and mixed with various reagents in the reaction cell R0. For example, in a case where the magnetic particles MB have a spherical shape, a diameter thereof is 0.1 to 10 µm, preferably 0.1 to 5 µm, and more preferably approximately 1 to 3 µm.

The cell R1 contains a buffer solution 36. The cell R2 accommodates a labeling reagent 37 containing the label S modified with a second binding substance B2 for specifically binding to the target substance. A first luminescent reagent 38 is contained in the cell R3, and a second luminescent reagent 39 is contained in the cell R4. In the present example, the label S is an enzyme, and the label S emits light in presence of the first luminescent reagent 38 and the second luminescent reagent 39. The labeling reagent 37 in the cell R2, the first luminescent reagent 38 in the cell R3, and the second luminescent reagent 39 in the cell R4 are simply referred to as reagents 37 to 39, in a case where it is not necessary to distinguish the respective liquids.

The first binding substance B1 and the second binding substance B2, which specifically bind to the target substance, are, for example, an antibody against an antigen in a case where the target substance is the antigen, an antigen against an antibody in a case where the target substance is the antibody, and an aptamer against a protein, a low-molecular-weight compound, or the like in a case where the target substance is the protein, the low-molecular-weight compound, or the like. The first binding substance B1 and the second binding substance B2 may be the same or different from each other.

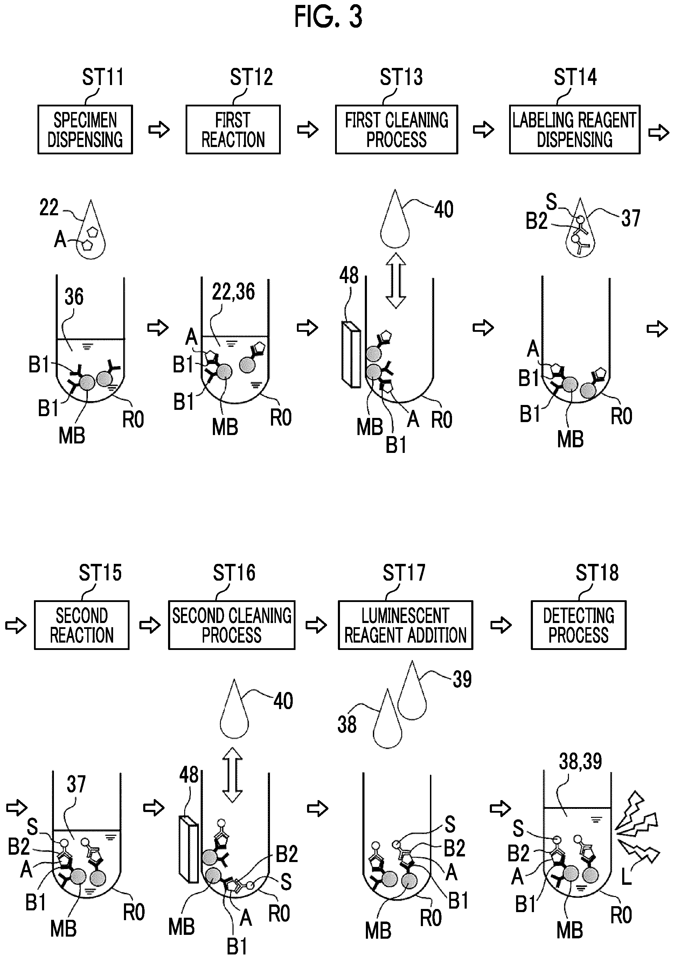

Here, a procedure of the measurement process according to the present example based on the chemiluminescent enzyme immunoassay method will be described with reference to Fig. 3. Fig. 3 schematically shows a reaction in a case where the specimen 22 contains the examination target substance A.

The reaction cell R0 contains the magnetic particles MB modified with the first binding substance B1 in advance, and a buffer solution 36 is dispensed into the reaction cell R0 before the dispensing of the specimen 22. In this state, the specimen 22 is dispensed into the reaction cell R0 (step ST11).

In the reaction cell R0, the magnetic particles MB, the specimen 22, and the buffer solution 36 are mixed with each other, and as a first reaction, a binding reaction in which the examination target substance A in the specimen 22 and the first binding substance B1 are specifically bound to each other occurs (step ST12). In the first reaction, the examination target substance A in the specimen 22 binds to the first binding substance B1, so that the examination target substance A is captured by the magnetic particles MB through the first binding substance B1.

Next, a first cleaning process (B/F separation) for removing unreacted components other than the examination target substance A, which are captured by the magnetic particles MB, is performed (step ST13). A magnet 48 is disposed close to an outside of the reaction cell R0, a reaction liquid after the first reaction is discharged in a state in which the magnetic particles MB are collected on an inner wall surface of the reaction cell R0, and a cleaning liquid 40 is dispensed into the reaction cell R0. In the step ST13 in Fig. 3, a bidirectional arrow in the up-down direction shown at the upper portion of the reaction cell R0 schematically indicates a state in which the cleaning liquid 40 is dispensed into the reaction cell R0 and discharged from the reaction cell R0. In the first cleaning process, the dispensing and the discharging of the cleaning liquid 40 are repeated a plurality of times. The cleaning liquid 40 is also discharged from the reaction cell R0 in a state in which the magnet 48 is disposed close to the outside of the reaction cell R0 and the magnetic particles MB are collected on the inner wall surface of the reaction cell R0.

After the first cleaning process, the labeling reagent 37 is dispensed into the reaction cell R0 (step ST14). The labeling reagent 37 contains a second binding substance B2 to which the label S is imparted, which is a binding substance for specifically binding to the examination target substance A.

In the reaction cell R0, as a second reaction, a binding reaction in which the examination target substance A captured by the magnetic particles MB and the second binding substance B2 are specifically bound to each other (step ST15). As a result, the label S is imparted to the examination target substance A through the second binding substance B2.

Next, a second cleaning process (B/F separation) for removing unreacted components other than the second binding substance B2 bound to the examination target substance A, which are captured by the magnetic particles MB in the labeling reagent 37, is performed (step ST16). The second cleaning process (step ST16) is performed in the same manner as the first cleaning process (step ST13).

Thereafter, the first luminescent reagent 38 and the second luminescent reagent 39 are added to the reaction cell R0 (step ST17). The label S is an enzyme, and causes a chemiluminescence L in the presence of the first luminescent reagent 38 and the second luminescent reagent 39, containing a luminescent substrate. The examination target substance A is detected by detecting the chemiluminescence L (step ST18). The procedure of the measurement process is as described above.

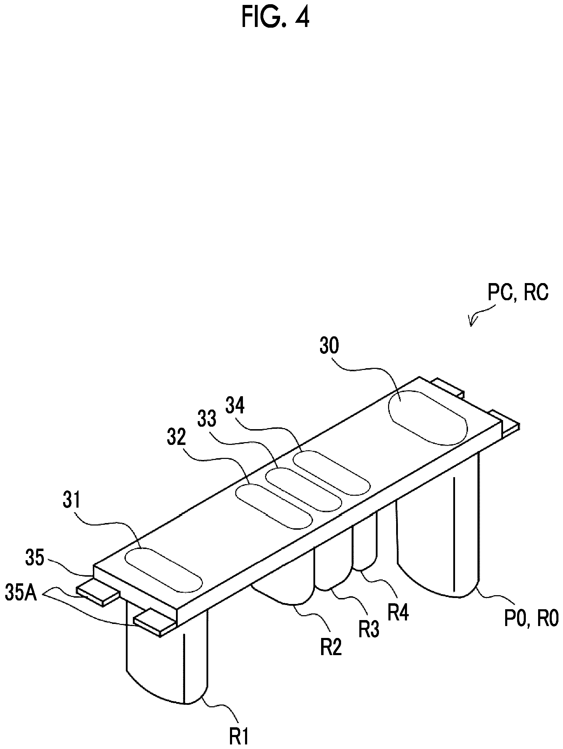

Depending on examination item, the specimen 22 may be pre-treated before the execution of the measurement process in order to improve accuracy of the examination. The pre-treatment is a treatment of heating the specimen 22 in a state of being mixed with a pre-treatment liquid. A cartridge PC for pre-treatment (see Fig. 4) different from the cartridge RC for measurement is used for the pre-treatment. As shown in Fig. 4, the cartridge PC for pre-treatment has a cell P0 for pre-treatment. The cell P0 for pre-treatment accommodates a mixed liquid in which the specimen 22 and a pre-treatment liquid are mixed with each other. In addition, a pre-treatment time which is a heating time for heating the specimen 22 mixed with the pre-treatment liquid is set to an appropriate time depending on the examination item. In the examination apparatus 10, as will be described later, the pre-treatment time is controlled for each examination item. As information for this, information related to the pre-treatment time such as the pre-treatment time for each examination item and a position on a transport passage 61 for pre-treatment (see Fig. 5) for transporting the cartridge PC for pre-treatment in accordance with the pre-treatment time is used. These pieces of information are stored in a memory 17 as setting information.

As shown in Fig. 4, the cartridge PC for pre-treatment in the present example has the same shape as the cartridge RC for measurement. The reaction cell R0 in the cartridge RC for measurement corresponds to the cell P0 for pre-treatment in the cartridge PC for pre-treatment. It is preferable that the cartridge PC for pre-treatment accommodates the pre-treatment liquid in at least one of the cells R1 to R4 before use. In the cartridge PC for pre-treatment, a plurality of cells among the cells R1 to R4 may accommodate different types of pre-treatment liquids. It is sufficient that the cartridge PC for pre-treatment has at least the cell P0 for pre-treatment, which accommodates the mixed liquid of the specimen 22 and the pre-treatment liquid, and has a shape which allows the cartridge PC for pre-treatment to be transported through the transport passage 61 for pre-treatment described later; and the cartridge PC for pre-treatment may not have the same shape as the cartridge RC for measurement.

As shown in Fig. 1, the examination apparatus 10 includes, as an example, a specimen transporting unit 11, a dispensing mechanism 12 as a specimen transfer mechanism, a pre-treatment line 13, a measurement line 14, a processor 16, a memory 17, and a touch panel display 18.

The specimen transporting unit 11 has a loading section (not shown) into which the specimen collection container 20 for accommodating the specimen 22 is loaded, and transports the loaded specimen collection container 20 to a position accessible by a nozzle 12A of the dispensing mechanism 12, which will be described later.

The processor 16 integrally controls each unit of the examination apparatus 10. An example of the processor 16 is a central processing unit (CPU) which performs various types of control by executing a program. The CPU functions as a control unit which controls each unit by executing a program.

In addition, the processor 16 acquires information on a light amount of the chemiluminescence L in a detecting unit 15 described later, and calculates a density of the examination target substance A based on the information on the light amount.

The memory 17 is an example of a memory connected to or built into the CPU as the processor 16. For example, the memory 17 stores a control program. In addition to the control program, the memory 17 stores setting information which is preset in order for the processor 16 to perform the various types of control.

In addition, the memory 17 stores information indicating a correspondence relationship between the light amount of the chemiluminescence L detected by the detecting unit 15 and the amount of the examination target substance A. The correspondence relationship is stored, for example, as a calibration curve represented by a function. The correspondence relationship may be a format of a table. The processor 16 calculates the amount of the examination target substance A from, for example, the light amount of the chemiluminescence L acquired from the detecting unit 15, and the calibration curve stored in the memory 17.

The touch panel display 18 receives, from a user, an operation instruction such as an instruction to start the examination. In addition, the touch panel display 18 displays information such as an examination result.

Fig. 5 is a schematic view showing an arrangement of the dispensing mechanism 12, the pre-treatment line 13, and the measurement line 14. In the drawing, a direction in which the transport passage 51 for measurement, which will be described later, of the measurement line 14 extends is referred to as an X direction, a direction orthogonal to the X direction in a horizontal plane is referred to as a Y direction, and a vertical direction perpendicular to an X-Y plane is referred to as a Z direction. The directions indicated by arrows X, Y, and Z in the following drawings match each other.

The measurement line 14 includes the transport mechanism 50 for measurement and various mechanisms used for the above-described measurement process. The transport mechanism 50 for measurement has the transport passage 51 for measurement, which linearly transports the cartridge RC for measurement in one direction (here, the X direction) from an upstream side to a downstream side. In the measurement line 14, the measurement process described with reference to Fig. 3 is executed while the cartridge RC for measurement is transported along the transport passage 51 for measurement.

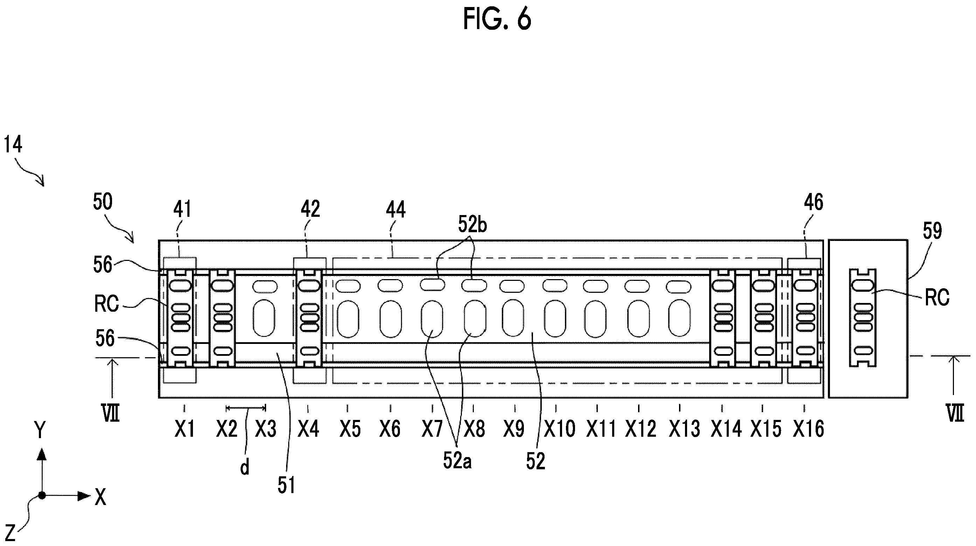

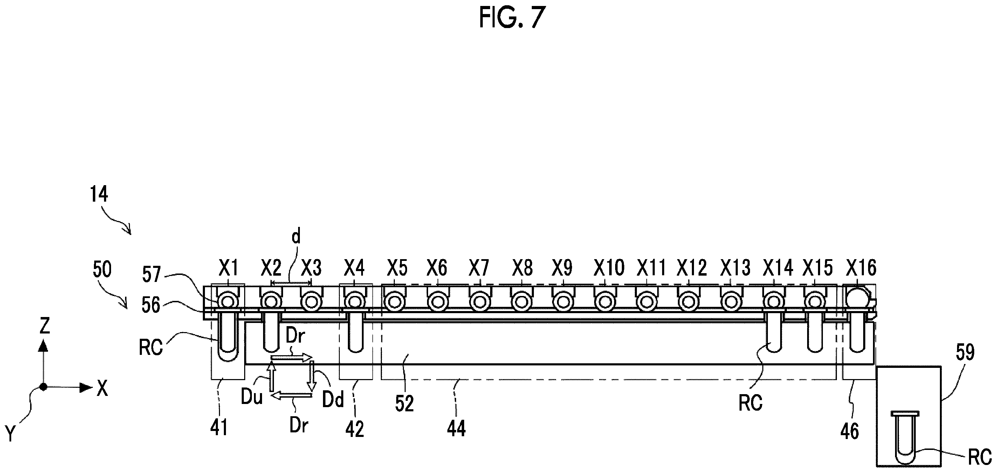

Fig. 6 is a plan view showing a schematic configuration of the transport passage 51 for measurement of the measurement line 14, and Fig. 7 is a cross-sectional view taken along a line VII-VII in Fig. 6.

A start end part of the transport passage 51 for measurement is a cartridge set position 41 at which the cartridge RC for measurement is set. The cartridge RC for measurement is transported to a terminal end on the downstream side of the transport passage 51 for measurement with the cartridge set position 41 as an upstream end in the transport direction. A specimen dispensing position 42 at which the specimen 22 is dispensed to the cartridge RC for measurement is provided on the downstream side of the cartridge set position 41 along the transport passage 51 for measurement. Furthermore, a processing section 44 and a detection position 46 are provided on the downstream side in this order. The detection position 46 is positioned at a terminal end part of the transport passage 51 for measurement. At the terminal end of the transport passage 51 for measurement, a cartridge discarding unit 59 that the cartridge RC for measurement, in which the measurement has been completed, is discarded is provided below in a vertical direction orthogonal to the transport direction. The cartridge RC for measurement is transported along the transport passage 51 for measurement, and is sequentially transported to the specimen dispensing position 42, the processing section 44, and the detection position 46. At the specimen dispensing position 42, specimen dispensing (step ST11) in the measurement process shown in Fig. 3 is performed. At the processing section 44, each piece of processes from first reaction (step ST12) to addition of a luminescent reagent (step ST17) in the measurement process shown in Fig. 3 is sequentially performed. At the detection position 46, detecting process (step ST18) of the measurement procedure shown in Fig. 3 is performed.

On the transport passage 51 for measurement of the measurement line 14, the processing section 44 includes a reagent dispensing mechanism (not shown) that includes a nozzle for suctioning the reagents 37 to 39 and dispensing each of the reagents into the reaction cell R0, and a cleaning nozzle for discharging the cleaning liquid 40 to the reaction cell R0 and suctioning and discharging the cleaning liquid 40 after the cleaning process. The reagent dispensing mechanism includes a moving mechanism and a suction-discharge mechanism, similarly to the dispensing mechanism 12 described later.

In addition, the measurement line 14 is provided with the detecting unit 15 (see Fig. 1) which detects the examination target substance in the specimen 22 from the cartridge RC for measurement disposed at the detection position 46 of the transport passage 51 for measurement. In the present example, the detecting unit 15 is composed of a photodetector such as a photomultiplier tube and a photodiode. The detecting unit 15 optically detects the examination target substance A bound to the label S by receiving the chemiluminescence L. For example, the detecting unit 15 is disposed to face the reaction cell R0 of the cartridge RC for measurement at the detection position, and receives the chemiluminescence L generated from the reaction solution in the reaction cell R0. The detecting unit 15 outputs a received light signal corresponding to an amount of received light to the processor 16.

The transport mechanism 50 for measurement includes a heat block 52 (see Fig. 6; not shown in Fig. 5) as a heating unit, a pair of guide parts 56 which are long in the transport direction for guiding the transport of the cartridge RC for measurement, and a plurality of rotating bodies 57 (see Fig. 7; not shown in Fig. 5, Fig. 6, and the like) which are arranged on each guide part 56 at regular intervals along the guide parts 56. In the present example, the transport passage 51 for measurement is a space defined by the guide part 56 on the side and the heat block 52 below. The cartridge RC for measurement is held by the transport mechanism 50 for measurement by engaging with the guide part 56, and is transported along the transport passage 51 for measurement. The transport mechanism 50 for measurement can transport the cartridge RC for measurement while heating the cells R2 to R4 and the reaction cell R0.

As shown in Fig. 7, the rotating bodies 57 are arranged at regular intervals of a distance d. As shown in Figs. 6 and 7, the X direction positions of the rotating bodies 57 arranged at regular intervals in the transport direction are represented by X1, X2, X3, ..., and X16 from the start end side of the transport passage 51 for measurement. The cartridge set position 41 is set to the position X1 at a start end of the transport passage 51 for measurement, and the specimen dispensing position 42 is set to the position X4. In addition, the processing section 44 is provided within a range from the position X5 to the position X15, and the detection position 46 is provided at the position X16.

Each of the guide parts 56 of the pair of guide parts 56 supports the flange portion 35A of the cartridge RC for measurement. As a result, the cartridge RC for measurement is suspended between the pair of guide parts 56. At each of the positions X1 to X16 where the rotating bodies 57 are provided, the flange portion 35A is sandwiched between the guide part 56 and the rotating body 57. In a case where the cartridge RC for measurement is moved, the rotating body 57 rotates with movement of the cartridge RC for measurement to assist the movement of the cartridge RC for measurement.

In addition, a plurality of sets of recess portions, in which a first recess portion 52a and a second recess portion 52b are set as one set, are provided in the heat block 52 along the transport direction. A disposition interval between the first recess portion 52a and the second recess portion 52b is the same distance d as the disposition interval of the rotating bodies 57 described above, and is the same as the disposition interval between the respective positions X1 to X16. The heat block 52 is one of constituent elements of the transport mechanism 50 for measurement, and the transport mechanism 50 for measurement includes a driving unit (not shown) which is an actuator for moving the heat block 52.

The heat block 52 heats the cells R2 to R4 and the reaction cell R0. In the transport passage 51 for measurement, the cartridge RC for measurement is set in a horizontal posture in which an arrangement direction of the cells R2 to R4 and the reaction cell R0 is orthogonal to the transport direction. One set of the first recess portion 52a and the second recess portion 52b is formed along the arrangement direction of the cells R2 to R4 and the reaction cell R0. In the cartridge RC for measurement, the cells R2 to R4 and the reaction cell R0 are inserted into the first recess portion 52a and the second recess portion 52b, and the cells R2 to R4 and the reaction cell R0 are heated by the insertion into the first recess portion 52a and the second recess portion 52b.

The cartridge RC for measurement, in a state in which the cells R2 to R4 are inserted into the first recess portion 52a and the reaction cell R0 is inserted into the second recess portion 52b, is transported by the transport passage 51 for measurement by moving the heat block 52 in the transport direction (that is, the X direction). The cartridge RC for measurement is supported by the guide part 56, but can be moved in the transport direction by the rotation of the rotating body 57. Therefore, in a case where the heat block 52 into which the cells R2 to R4 and the like are inserted, which is a part of the cartridge RC for measurement, is moved in the transport direction, the cartridge RC for measurement in a state of being supported by the guide part 56 is moved in the transport direction. In practice, a plurality of cartridges RC for measurement are held in the heat block 52. In this state, the heat block 52 is moved, and thus the plurality of cartridges RC for measurement are collectively transported in the transport direction.

The heat block 52 is connected to a heater (not shown) and is temperature-controlled by a temperature controller which controls the heater. A temperature of the heat block 52 is set to approximately 36°C to 38°C, preferably 37°C, from the viewpoint of promoting an immunoreaction.

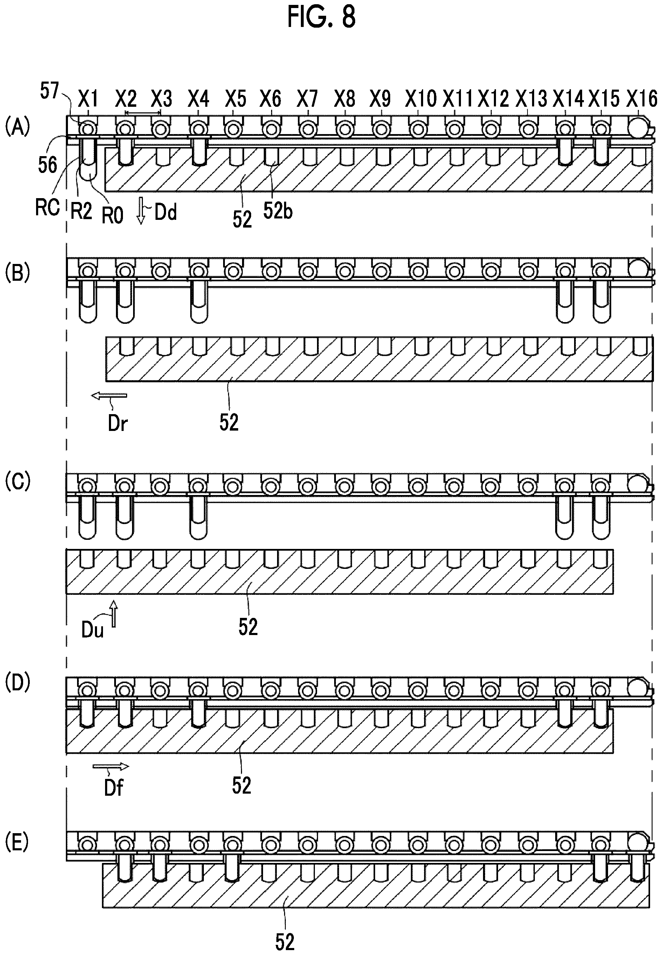

Fig. 8 schematically shows a transporting method using the heat block 52. The driving unit of the transport mechanism 50 for measurement can move the heat block 52 along four directions of arrows Dd, Dr, Du, and Df shown in Fig. 8 (see also Fig. 6). The movement along the arrow Dd is movement downward in the vertical direction, and the movement along the arrow Dr is movement upstream in the transport direction (that is, the X direction) in the horizontal direction. The movement along the arrow Du is movement upward in the vertical direction, and the movement along the arrow Df is movement downstream in the transport direction (that is, the X direction) in the horizontal direction. In a case where the operation of moving the cartridge RC for measurement in the four directions of the arrows Dd, Dr, Du, and Df in this order is set as a single period operation of the heat block 52, the cartridge RC for measurement is moved by the distance d in the transport direction with the single period operation of the heat block 52. A moving distance in the ±X direction along the arrows Dr and Df is the same distance d as the arrangement period of the rotating bodies 57. Therefore, for example, the cartridge RC for measurement at the position X1 is moved to the position X2 adjacent to the downstream side of the position X1 with the single period operation of the heat block 52.

The transporting method using the heat block 52 will be described in more detail with reference to Fig. 8. Fig. 8 shows a cross section along the transport direction, which is a cross section in which the cell R2 of the cartridge RC for measurement inserted into the first recess portion 52a and the second recess portion 52b is visible. First, (A) of Fig. 8 shows a state in which the cells R2 to R4 and the reaction cell R0 of the cartridge RC for measurement are inserted into the first recess portion 52a and the second recess portion 52b of the heat block 52 shown in Fig. 6, respectively. In this way, the heat block 52 is moved downward along the arrow Dd from the state in which the cartridge RC for measurement is inserted into the heat block 52. (B) of Fig. 8 shows a state in which the heat block 52 is moved downward. From the state of (B) of Fig. 8, the heat block 52 is moved by the distance d to the upstream side in the transport direction along the arrow Dr. (C) of Fig. 8 shows a state in which the heat block 52 is moved by the distance d to the upstream side in the transport direction. The heat block 52 is moved upward along the arrow Du from the state of (C) in Fig. 8. (D) of Fig. 8 shows a state in which the heat block 52 is moved upward. In (A) of Fig. 8, the cartridge RC for measurement at the position X1, which is not inserted into the heat block 52, is inserted into the heat block 52 in (D) of Fig. 8. From the state of (D) in Fig. 8, the heat block 52 is moved by the distance d in the transport direction along the arrow Df. As a result, as shown in (E) of Fig. 8, the cartridge RC for measurement at the position X1 in (A) of Fig. 8 is moved to the position X2.

In this way, the cartridge RC for measurement is transported from the position X1 corresponding to the cartridge set position 41 to the position X16 corresponding to the detection position 46, by repeating the movement of the heat block 52 by the single period operation in the directions of the arrows Dd, Dr, Du, and Df in the transport direction. After the detecting process is performed at the position X16 corresponding to the detection position 46, the cartridge RC for measurement is pushed out from the transport passage 51 for measurement with the movement of the heat block 52, falls downward from the terminal end of the transport passage 51 for measurement, and is accommodated in the cartridge discarding unit 59.

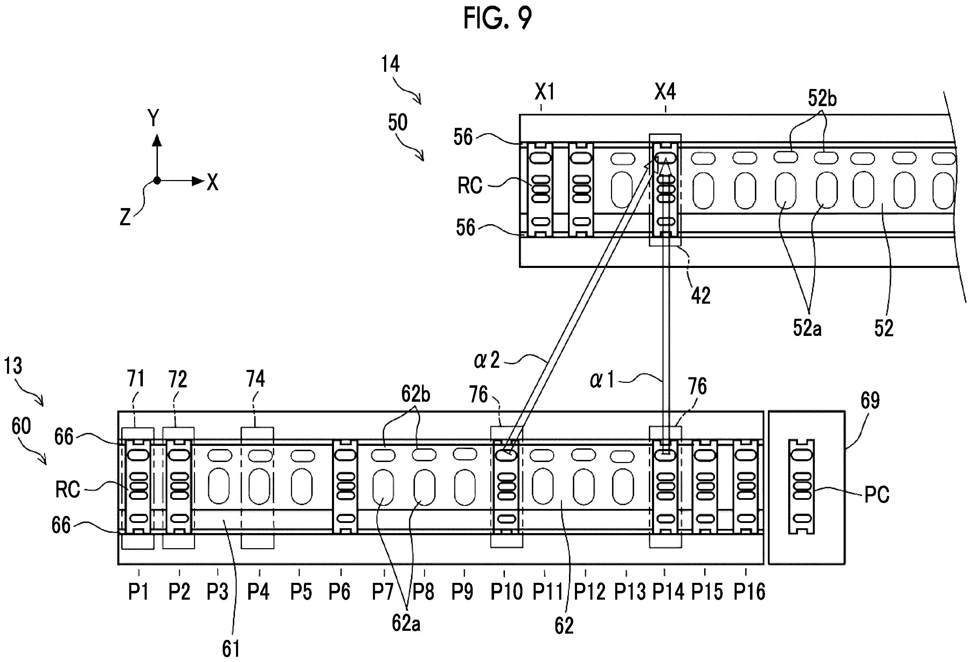

Fig. 9 is a plan view schematically showing the pre-treatment line 13 and the measurement line 14. The pre-treatment line 13 includes a transport mechanism 60 for pre-treatment. The transport mechanism 60 for pre-treatment has a transport passage 61 for pre-treatment for transporting the cartridge PC for pre-treatment used in the pre-treatment, which is different from the cartridge RC for measurement. In the pre-treatment line 13, the pre-treatment is executed while the cartridge PC for pre-treatment is transported along the transport passage 61 for pre-treatment. As shown in Fig. 9, the transport passage 61 for pre-treatment in the present example is a transport passage that linearly transports the cartridge PC for pre-treatment in one direction from an upstream side to a downstream side, similarly to the transport passage 51 for measurement.

In the present example, the configuration of the transport mechanism 60 for pre-treatment is substantially the same as that of the transport mechanism 50 for measurement. The transport mechanism 60 for pre-treatment includes a heat block 62 as a heating unit, a pair of guide parts 66 which are long in the transport direction for guiding the transport of the cartridge PC for pre-treatment, and a plurality of rotating bodies (not shown) which are arranged on each guide part 66 at regular intervals along the guide parts 66. The transport mechanism 60 for pre-treatment includes a driving unit (not shown) which is an actuator for moving the heat block 62. In the present example, the transport passage 61 for pre-treatment is a space defined by the guide part 66 on the side and the heat block 62 below. The cartridge PC for pre-treatment is held by the transport mechanism 60 for pre-treatment by engaging with the guide part 66, and is transported along the transport passage 61 for pre-treatment. The transport mechanism 60 for pre-treatment can transport the cartridge PC for pre-treatment while heating at least the cell P0 for pre-treatment. Same as the heat block 52, the heat block 62 is also connected to a heater (not shown) and is temperature-controlled by a temperature controller which controls the heater. In the present example, the heat block 52 in the measurement line 14 and the heat block 62 in the pre-treatment line 13 are connected to heaters different from each other, and the temperatures can be set independently. That is, the heating units (the heat block 52 and the heat block 62) of the measurement line 14 and the pre-treatment line 13 can be controlled to different temperatures. The heat block 62 is set to a temperature suitable for the pre-treatment, which promotes a reaction between the specimen 22 and the pre-treatment liquid. The function of each constituent element of the transport mechanism 60 for pre-treatment is the same as the function of each constituent element of the transport mechanism 50 for measurement. Therefore, in a case where the heat block 62 in which the cells R2 to R4 and the cell P0 for pre-treatment are inserted, which is a part of the cartridge PC for pre-treatment, is moved in the transport direction, the cartridge PC for pre-treatment in a state of being supported by the guide part 66 is moved in the transport direction. The transport mechanism 60 for pre-treatment can transport the cartridge PC for pre-treatment while heating the cell P0 for pre-treatment.

As shown in Fig. 9, in the transport passage 61 for pre-treatment, transport direction positions of the rotating bodies arranged at regular intervals in the transport direction are represented by P1, P2, P3, ..., and P16 from the start end side. In the present example, a cartridge set position 71 at which the cartridge PC for pre-treatment is set at the position P1 of a start end part of the transport passage 61 for pre-treatment is provided. A pre-treatment liquid dispensing position 72 for dispensing the pre-treatment liquid into the cell P0 for pre-treatment is provided at the position P2 on the downstream side of the cartridge set position 71 along the transport passage 61 for pre-treatment. Furthermore, a specimen dispensing position 74 at which the specimen 22 is dispensed into the cartridge PC for pre-treatment is provided at the position P4 on the downstream side of the pre-treatment liquid dispensing position 72. The cartridge PC for pre-treatment is transported to a terminal end on the downstream side of the transport passage 61 for pre-treatment with the cartridge set position 71 as an upstream end in the transport direction, through the pre-treatment liquid dispensing position 72 and the specimen dispensing position 74. At the terminal end of the transport passage 61 for pre-treatment, a cartridge discarding unit 69 that the cartridge PC for pre-treatment is discarded is provided below in a vertical direction orthogonal to the transport direction.

In the pre-treatment line 13, a pre-treatment liquid dispensing mechanism (not shown) including a nozzle for the pre-treatment liquid is provided on the transport passage 61 for pre-treatment. At the pre-treatment liquid dispensing position 72, the pre-treatment liquid accommodated in any of the cells R1 to R4 is suctioned and discharged to the cell P0 for pre-treatment. The pre-treatment liquid dispensing mechanism includes a moving mechanism and a suction-discharge mechanism, similarly to the dispensing mechanism 12 described later.

At the specimen dispensing position 74, the cartridge PC for pre-treatment is transported downstream along the transport passage 61 for pre-treatment in a state in which the specimen 22 and the pre-treatment liquid are mixed in the cell P0 for pre-treatment, the specimen 22 is mixed with the pre-treatment liquid, the cell P0 for pre-treatment is inserted into a second recess portion 62b of the heat block 62, and the cells R2 to R4 are inserted into a first recess portion 62a. During the transport, the specimen 22 mixed with the pre-treatment liquid accommodated in the cell P0 for pre-treatment is heated by the heat block 62. In the present example, the heating is started from the specimen dispensing position 74 at which the specimen 22 is dispensed to the cell P0 for pre-treatment in a state in which the pre-treatment liquid and the specimen 22 are mixed with each other. That is, the specimen dispensing position 74 corresponds to the pre-treatment start position.

Each of the cartridges PC for pre-treatment is transported for a pre-treatment time according to the examination item of the specimen 22. At the pre-treatment end position 76 transported at the end of the pre-treatment time according to the examination item, the pre-treated specimen 22 in which the pre-treatment is completed is transferred to the cartridge for measurement by a specimen transfer mechanism described later.

In Fig. 9, the pre-treatment end position 76 is illustrated at the position P14, as an example. However, as described above, there is an appropriate pre-treatment time depending on the examination item, and the pre-treatment end position 76 on the transport passage 61 for pre-treatment varies depending on the pre-treatment time. The pre-treatment end position 76 is any position from the position P5 to the position P16, and is determined by the examination item. The pre-treatment time is set to a time suitable for each examination item, that is, each type of the target substance to be detected. Since the cartridge PC for pre-treatment is transported for a predetermined pre-treatment time depending on detection item, the pre-treatment end position 76 is determined depending on the detection item. The pre-treatment time for each examination item or information related to the pre-treatment time is stored in the memory 17 in advance, and the processor 16 reads out the pre-treatment time corresponding to the examination item from the memory 17 at the time of the examination, and controls the transport mechanism 60 for pre-treatment such that the cartridge PC for pre-treatment is transported in the set pre-treatment time. After the pre-treatment is completed, the cartridge PC for pre-treatment in which the pre-treated specimen 22 is suctioned at the pre-treatment end position 76 is transported along the transport passage 61 for pre-treatment, is finally extruded from the terminal end of the transport passage 61 for pre-treatment, falls down, and is accommodated in the cartridge discarding unit 69.

As shown in Fig. 9, the transport passage 61 for pre-treatment and the transport passage 51 for measurement are arranged such that the pre-treatment end position 76 in the transport passage 61 for pre-treatment, at which the pre-treatment is completed, is closer to an upstream side in a transport direction of the transport passage 51 for measurement than a downstream side in the transport direction. In the present example, the pre-treatment end position 76 of the transport passage 61 for pre-treatment is any position from the position P5 to the position P16, and any position from the position P5 to the position P16 is disposed in an aspect closer to the upstream side in the transport direction of the transport passage 51 for measurement than the downstream side in the transport direction. The transport passage 61 for pre-treatment and the transport passage 51 for measurement are arranged such that the transport directions are parallel to each other and the downstream side of the transport passage 61 for pre-treatment and the upstream side of the transport passage 51 for measurement partially overlap each other. In the present specification, the upstream side and the downstream side in the transport passage 51 for measurement are defined such that a half position (center position) of the transport passage 51 for measurement is set to a reference, and the upstream side is a side closer to the start end than the reference and the downstream side is a side closer to the terminal end than the reference. In the example shown in Fig. 7, the positions X1 to X8 are on the upstream side, and the positions X9 to X16 are on the downstream side. Similarly, the upstream side and the downstream side in the transport passage 61 for pre-treatment are defined such that a half position (center position) of the transport passage 61 for pre-treatment is set to a reference, and the upstream side is a side closer to the start end than the reference and the downstream side is a side closer to the terminal end than the reference. In the example shown in Fig. 9, the positions P1 to X8 are on the upstream side, and the positions P9 to P16 are on the downstream side.

The dispensing mechanism 12 is an example of the specimen transfer mechanism that transfers the pre-treated specimen 22 in which the pre-treatment has been completed, from the cartridge PC for pre-treatment in the pre-treatment line 13 to the cartridge RC for measurement in the measurement line 14.

The dispensing mechanism 12 is a mechanism which dispenses a liquid, and includes a nozzle 12A which suctions and discharges the specimen 22, a nozzle moving mechanism 24 (see Fig. 5), and a suction-discharge mechanism (not shown). The nozzle 12A is provided with a replaceable tip at a distal end thereof. The nozzle moving mechanism 24 includes an X direction movable part (not shown), a Y direction movable part 24B, and a Z direction movable part 24C, and is a mechanism that three-dimensionally moves the nozzle 12A in a vertical direction (Z direction) and a horizontal direction (X-Y direction). The suction-discharge mechanism is a mechanism that causes the nozzle 12A to perform a suction operation of suctioning the liquid and a discharge operation of discharging the liquid from the nozzle 12A. The nozzle moving mechanism 24 includes an actuator such as a motor, which generates a driving force, and a driving force transmission mechanism consisting of a gear, a drive belt, and the like. The suction-discharge mechanism is composed of a pump or the like, which generates a driving force for the suction and discharge.

As shown in Fig. 5, the dispensing mechanism 12 suctions the pre-treated specimen 22 from the cell P0 for pre-treatment of the cartridge PC for pre-treatment at any position (for example, the position P14) on the transport passage 61 for pre-treatment (arrow α11). The dispensing mechanism 12 moves the nozzle 12A to the specimen dispensing position X4 on the transport passage 51 for measurement (arrow α12),and discharges the pre-treated specimen 22 to the reaction cell R0 of the cartridge RC for measurement, positioned at the specimen dispensing position X4 (arrow α13). Fig. 5 shows a configuration in which the pre-treated specimen 22 is transferred from the position P14 on the transport passage 61 for pre-treatment, closest to the specimen dispensing position X4 on the transport passage 51 for measurement. The dispensing mechanism 12 can transfer the pre-treated specimen 22 by transferring along the arrows α11 to α13 described in Fig. 5, as shown by the arrow α1 in Fig. 9, and can also transfer the pre-treated specimen 22 from the cartridge PC for pre-treatment, which is at any pre-treatment end position 76 (in the present example, any position from the position P5 to the position P16) of the transport passage 61 for pre-treatment, such as the position P10, to the cartridge RC for measurement, which is at the specimen dispensing position 42 on the transport passage 51 for measurement, as shown by the arrow α2.

The dispensing mechanism 12 may have a function of suctioning the specimen 22 from the specimen collection container 20 and dispensing the specimen 22 to the cell P0 for pre-treatment of the cartridge PC for pre-treatment. In addition, in a case where it is not necessary to perform the pre-treatment on the specimen 22, the dispensing mechanism 12 may suction the specimen 22 from the specimen collection container 20 and dispense the specimen 22 to the reaction cell R0 of the cartridge RC for measurement. As described above, the dispensing mechanism 12 may also serve as a specimen transfer mechanism and a specimen dispensing mechanism.

The action of the above-described configuration will be described. Examination of a case in which the specimen 22 on which the pre-treatment has been performed is subjected to the measurement process will be described.

A specimen collection container 20 which accommodates the specimen 22 to be examined is loaded into the examination apparatus 10. In the examination apparatus 10, a plurality of the cartridges PC for pre-treatment and a plurality of the cartridges RC for measurement are set in a loading section (not shown) in advance.

In a case where examination order for the specimen 22 requiring the pre-treatment is received, the processor 16 controls a pre-treatment cartridge set mechanism (not shown) to set the specimen 22 at the cartridge set position 71 of the transport passage 61 for pre-treatment. In the transport mechanism 60 for pre-treatment, the heat block 62 is operated at a constant cycle to intermittently transport the cartridge on the transport passage 61 for pre-treatment in the transport direction by the distance d.

Next, the processor 16 controls the pre-treatment liquid dispensing mechanism (not shown) to dispense the pre-treatment liquid to be mixed with the specimen 22 to the cell P0 for pre-treatment of the cartridge PC for pre-treatment at the pre-treatment liquid dispensing position 72. The pre-treatment liquid is accommodated in, for example, the cell R1 in the cartridge PC for pre-treatment, and the pre-treatment liquid in the cell R1 is dispensed into the cell P0 for pre-treatment by the reagent dispensing mechanism including a nozzle for dispensing the pre-treatment liquid.

Thereafter, the processor 16 controls the dispensing mechanism 12 to suction the specimen 22 accommodated in the specimen collection container 20 at the specimen dispensing position 74 by the nozzle 12A, and to dispense the specimen 22 into the cell P0 for pre-treatment. In this case, the suction and discharge operation is repeated in the cell P0 for pre-treatment by the nozzle 12A, and thus the specimen 22 and the pre-treatment liquid are mixed with each other.

Thereafter, the cartridge PC for pre-treatment is transported to the downstream side along the transport passage 61 for pre-treatment. In this case, the cell P0 for pre-treatment is transported in a state in which the heat block 62 is inserted into the second recess portion 62b. Therefore, while the cartridge PC for pre-treatment is transported, the specimen 22 mixed with the pre-treatment reagent in the cell P0 for pre-treatment is heated by the heat block 62, and is subjected to the pre-treatment.

The processor 16 transports the cartridge PC for pre-treatment to any position according to the pre-treatment time determined for each examination item by the transport mechanism 60 for pre-treatment. As an example, the cartridge PC for pre-treatment is transported to the pre-treatment end position 76 at the position P14 shown in Fig. 9.

The processor 16 controls the dispensing mechanism 12 such that the nozzle 12A suctions the pre-treated specimen 22 from the cell P0 for pre-treatment of the cartridge PC for pre-treatment at the position P14 in the transport passage 61 for pre-treatment. Next, the processor 16 moves the nozzle 12A from the pre-treatment end position 76 to the position X4 corresponding to the specimen dispensing position 42 of the transport passage 51 for measurement, and dispenses the pre-treated specimen 22 to the reaction cell R0 of the cartridge RC for measurement at the specimen dispensing position 42.

The processor 16 controls a cartridge set mechanism (not shown) to set the cartridge RC for measurement at the cartridge set position 41 of the transport passage 51 for measurement during the transporting of the cartridge PC for pre-treatment. In the present example, in a case where the cartridge PC for pre-treatment is transported to the pre-treatment end position 76, the cartridge RC for measurement is set at the cartridge set position 41 at a timing at which the cartridge RC for measurement is transported to the specimen dispensing position 42.

The cartridge PC for pre-treatment, having the cell P0 for pre-treatment from which the pre-treated specimen 22 is suctioned, is transported to the downstream side as it is, and is pushed out from the transport passage 61 for pre-treatment at the terminal end of the transport passage 61 for pre-treatment, falls down, and is accommodated in the cartridge discarding unit 69.

On the other hand, the cartridge RC for measurement, having the reaction cell R0 in which the pre-treated specimen 22 is dispensed, is sequentially transported along the transport passage 51 for measurement, the processing section 44, and the detection position 46. In the processing section 44, each piece of processes from the first reaction (step ST12) to the addition of the luminescent reagent (step ST17) in the measurement process shown in Fig. 3 is sequentially performed, and the detecting process (step ST18) in the measurement process shown in Fig. 3 is performed at the detection position 46.

Similarly to the cartridge PC for pre-treatment, the cartridge RC for measurement is pushed out from the transport passage 51 for measurement at the terminal end of the transport passage 51 for measurement, falls down, and is accommodated in the cartridge discarding unit 59.

As described above, the examination apparatus 10, which is an example of the embodiment, includes the measurement line 14 that performs a measurement process while transporting the cartridge RC for measurement along transport passage 51 for measurement, the pre-treatment line 13 that performs a pre-treatment while transporting the cartridge PC for pre-treatment along the transport passage 61 for pre-treatment, and a specimen transfer mechanism (here, the dispensing mechanism 12) that transfers the pre-treated specimen 22 in which the pre-treatment is completed from the cartridge PC for pre-treatment to the cartridge RC for measurement. The transport passage 61 for pre-treatment and the transport passage 51 for measurement are arranged such that the pre-treatment end position 76 in the transport passage 61 for pre-treatment, at which the pre-treatment is completed, is closer to an upstream side in a transport direction of the transport passage 51 for measurement than a downstream side in the transport direction. By including the pre-treatment line 13, the pre-treatment can be executed in the examination apparatus 10. With the present configuration, for example, the pre-treatment end position 76 and the specimen dispensing position 42 in the transport passage 51 for measurement can be arranged close to each other, as compared with a case in which the transport passage 51 for measurement and the transport passage 61 for pre-treatment are arranged to be adjacent to each other on the upstream side and the downstream side. Therefore, a transfer distance of the pre-treated specimen 22 can be set to be short, and occurrence of contamination due to the transfer of the pre-treated specimen 22 can be suppressed. Since the specimen transfer mechanism (here, the dispensing mechanism 12) that transfers the pre-treated specimen 22 from the cartridge PC for pre-treatment to the cartridge RC for measurement is provided, the pre-treatment can be performed for each examination item (examination target substance) in a suitable pre-treatment time.

In the present example, the transport passage 61 for pre-treatment is linear. In a case where the cartridge PC as shown in Fig. 4 is used, a linear shape may be more advantageous than a rotating type transport passage.

In the present example, the transport passage 61 for pre-treatment and the transport passage 51 for measurement are arranged such that the transport directions are parallel to each other and the downstream side of the transport passage 61 for pre-treatment and the upstream side of the transport passage 51 for measurement partially overlap each other. With such an arrangement, the pre-treatment end position 76 and the specimen dispensing position 42 in the transport passage 51 for measurement can be brought close to each other, and the occurrence of contamination due to the transfer of the specimen 22 can be suppressed. In addition, it is possible to improve transfer efficiency of the pre-treated specimen 22.

It is preferable that the transport passage 61 for pre-treatment and the transport passage 51 for measurement are arranged such that the downstream side in a transport direction of the transport passage 61 for pre-treatment is closest to the upstream side in the transport direction of the transport passage 51 for measurement. The aspect in which the transport passage 61 for pre-treatment and the transport passage 51 for measurement are arranged such that the downstream side of the transport passage 61 for pre-treatment in the transport direction and the upstream side of the transport passage 51 for measurement in the transport direction are closest to each other refers to a state in which at least a part of the downstream side of the transport passage 61 for pre-treatment in the transport direction is disposed to be close to at least a part of the upstream side of the transport passage 51 for measurement in the transport direction. The arrangement of the transport passage 61 for pre-treatment and the transport passage 51 for measurement shown in Figs. 5 and 9 is an example of this aspect. In such an arrangement, in a case where the pre-treatment is completed in a state in which the cartridge for treatment is positioned on the downstream side of the transport passage for pre-treatment and the measurement process is started in a state in which the cartridge for measurement is positioned on the upstream side of the transport passage for measurement process, the transfer distance of the pre-treated specimen can be shortened, and the concern of the contamination due to the transfer of the specimen can be further suppressed.

In addition, in the present example, the measurement line 14 and the pre-treatment line 13 each have a heating unit (here, the heat block 52 and the heat block 62) for heating the specimen 22, and each heating unit is controllable to a different temperature. In a case where the measurement line is also used for the pre-treatment without providing the pre-treatment line, the temperature for heating the cell P0 for pre-treatment of the cartridge PC for pre-treatment cannot be set to a temperature different from the temperature for heating the reaction cell R0 of the cartridge RC for measurement. However, according to the present example, since the heating temperature in the pre-treatment line 13 can be controlled to a temperature different from the heating temperature in the measurement line 14, the pre-treatment can be performed at an appropriate temperature according to the examination item.

In the present example, the specimen transfer mechanism is a dispensing mechanism 12 that suctions the pre-treated specimen 22 from the cartridge PC for pre-treatment and discharges the pre-treated specimen 22 to the cartridge RC for measurement. It is preferable that the dispensing mechanism 12 is capable of suctioning the pre-treated specimen 22 from the cartridge PC for pre-treatment at any position on the transport passage 61 for pre-treatment. With the configuration, it is possible to perform the pre-treatment with an appropriate pre-treatment time for each examination item.

In the present example, the dispensing mechanism 12 is also used for dispensing the specimen 22 which has not been subjected to the pre-treatment into the reaction cell R0, in addition to transferring the pre-treated specimen 22 from the cell P0 for pre-treatment to the reaction cell R0. However, a dispensing mechanism as a specimen transfer mechanism which transfers the pre-treated specimen 22 from the cell P0 for pre-treatment to the reaction cell R0, and a specimen dispensing mechanism which dispenses the un-treated specimen 22 to the reaction cell R0 may be separately provided. In this case, the specimen dispensing mechanism is used not only for dispensing the specimen 22, which does not need the pre-treatment, to the reaction cell R0, but also for dispensing the specimen 22, which needs the pre-treatment, to the cell P0 for pre-treatment.

However, as in the present example, in a case where the dispensing mechanism 12 is also used for dispensing the specimen 22, which has not been subjected to the pre-treatment, into the reaction cell R0, in addition to transferring the specimen 22, which has been subjected to the pre-treatment, from the cell P0 for pre-treatment to the reaction cell R0, the examination apparatus can be configured to be smaller than in a case where two dispensing mechanisms are provided.

In the above-described example, the pre-treatment liquid and the specimen 22 are respectively dispensed to the cell P0 for pre-treatment at the pre-treatment liquid dispensing position 72 and the specimen dispensing position 74 on the transport passage 61 for pre-treatment. However, the cartridge PC for pre-treatment may be configured to be set at the cartridge set position 71 in a state in which the pre-treatment liquid and the specimen 22 are mixed in advance in the cell P0 for pre-treatment. In a case where the cartridge PC for pre-treatment is set at the cartridge set position 71 in a state in which the pre-treatment liquid and the specimen 22 are mixed in advance in the cell P0 for pre-treatment, the specimen 22 mixed with the pre-treatment liquid is heated from the cartridge set position 71. That is, in a case where the cartridge is set in a state in which the pre-treatment liquid and the specimen 22 are mixed with each other, the cartridge set position 71 is the pre-treatment start position.

In the present example, the shape of the cartridge PC for pre-treatment is the same as the shape of the cartridge RC for measurement, but the shape of the cartridge PC for pre-treatment may be any shape as long as the cartridge PC for pre-treatment has at least the cell P0 for pre-treatment. It is sufficient that the transport mechanism 60 for pre-treatment is configured such that the cartridge PC for pre-treatment can be transported, and the transport mechanism 60 for pre-treatment may be configured according to the shape of the cartridge PC for pre-treatment.

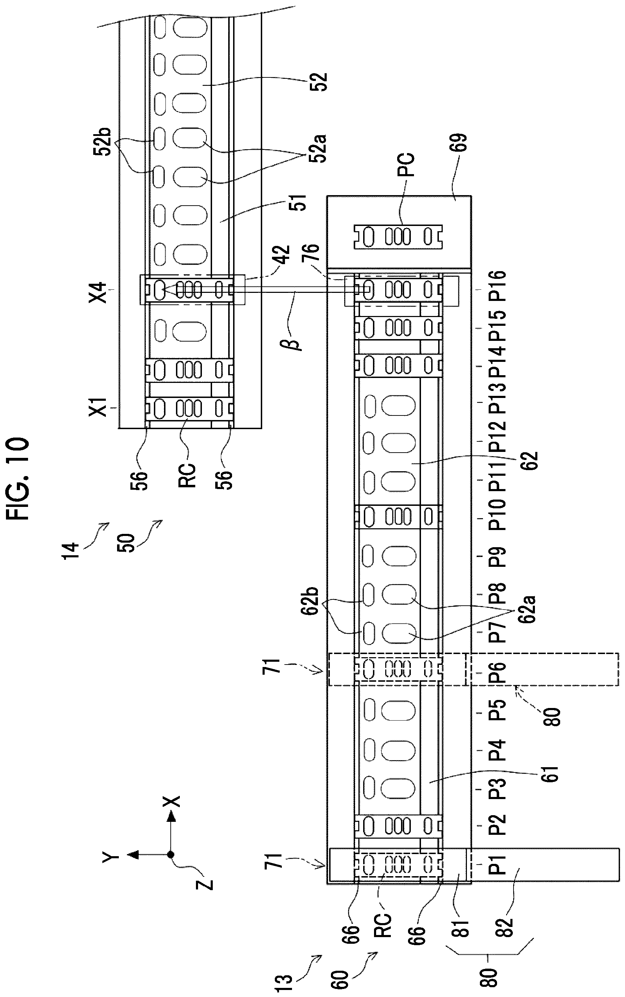

In addition, in the above-described example, the cartridge set position 71 is fixed at the position P1 in the transport passage 61 for pre-treatment, and the pre-treatment is performed for the pre-treatment time according to the examination item by making the pre-treatment end position 76 optional. On the other hand, as shown in Fig. 10, in the transport passage 61 for pre-treatment, the cartridge set position 71 may be variable and the pre-treatment end position 76 may be fixed. In Fig. 10, the cartridge set position 71 is set to the position P1 and the position P6. The pre-treatment end position 76 is set to the position P16 of the terminal end of the transport passage 61 for pre-treatment. In this case, in a case of transferring the pre-treated specimen 22, the dispensing mechanism 12 always suctions the pre-treated specimen 22 from the cartridge PC for pre-treatment transported to the pre-treatment end position 76. Next, the dispensing mechanism 12 transfers the pre-treated specimen 22 to the cartridge RC for measurement at the specimen dispensing position 42 set at the position X4 on the transport passage 51 for measurement, from the pre-treatment end position 76 set at the position P16 (see arrow β).

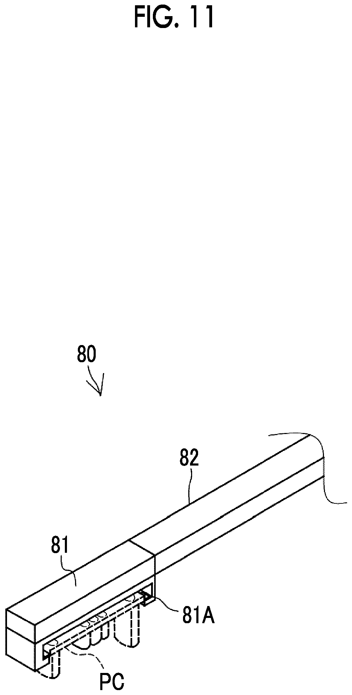

In this case, as the cartridge set mechanism of the cartridge PC for pre-treatment, a pre-treatment cartridge set mechanism 80 capable of setting the cartridge PC for pre-treatment at any position of the transport passage 61 for pre-treatment is provided. For example, as shown in Figs. 10 and 11, the pre-treatment cartridge set mechanism 80 includes a cartridge support part 81 and a moving mechanism 82 which moves the cartridge support part 81. As shown in Fig. 11, the cartridge support part 81 includes a pair of engaging portions 81A which engage with the pair of flange portions 35A of the cartridge PC for pre-treatment, and suspends the cartridge PC for pre-treatment between the pair of engaging portions 81A by supporting the flange portions 35A of the cartridge PC for pre-treatment with the engaging portions 81A. Although not shown in detail, the moving mechanism 82 is configured to move the cartridge support part 81 in the transport direction of the transport passage 61 for pre-treatment, in a direction orthogonal to the transport direction in a horizontal plane and in a vertical direction (Z direction). The moving mechanism 82 includes an actuator such as a motor, which generates a driving force, and a driving force transmission mechanism consisting of a gear, a drive belt, and the like.

In this way, in the transport passage 61 for pre-treatment, in a case where at least one of the cartridge set position 71 or the pre-treatment end position 76 is variable, the pre-treatment time can be adjusted, and the pre-treatment can be performed for an appropriate pre-treatment time according to the examination item. A configuration may be adopted in which the pre-treatment time can be adjusted by making both the cartridge set position 71 and the pre-treatment end position 76 variable. As described above, since the specimen dispensing position 74 set at the cartridge set position 71 or in the vicinity of the cartridge set position 71 is the pre-treatment start position, the cartridge set position 71 being variable is synonymous with the pre-treatment start position being variable.

As shown in Fig. 10, in a case where the pre-treatment end position 76 in the transport passage 61 for pre-treatment and the specimen dispensing position 42 in the transport passage 51 for measurement are fixed, it is preferable that the transport passage 61 for pre-treatment and the transport passage 51 for measurement are arranged such that the pre-treatment end position 76 and the specimen dispensing position 42 are positioned closest to each other. This is because the transfer distance of the pre-treated specimen 22 can be set to be the shortest, and thus, the contamination due to the transfer can be suppressed, and the transfer can be efficiently performed. In the example shown in Fig. 10, the transport passage 61 for pre-treatment and the transport passage 51 for measurement are arranged in an aspect of being parallel and partially overlap, such that the position P16 which is the pre-treatment end position 76 of the transport passage 61 for pre-treatment is adjacent to the specimen dispensing position X4 of the transport passage 51 for measurement.

### "Examination apparatus according to second embodiment"

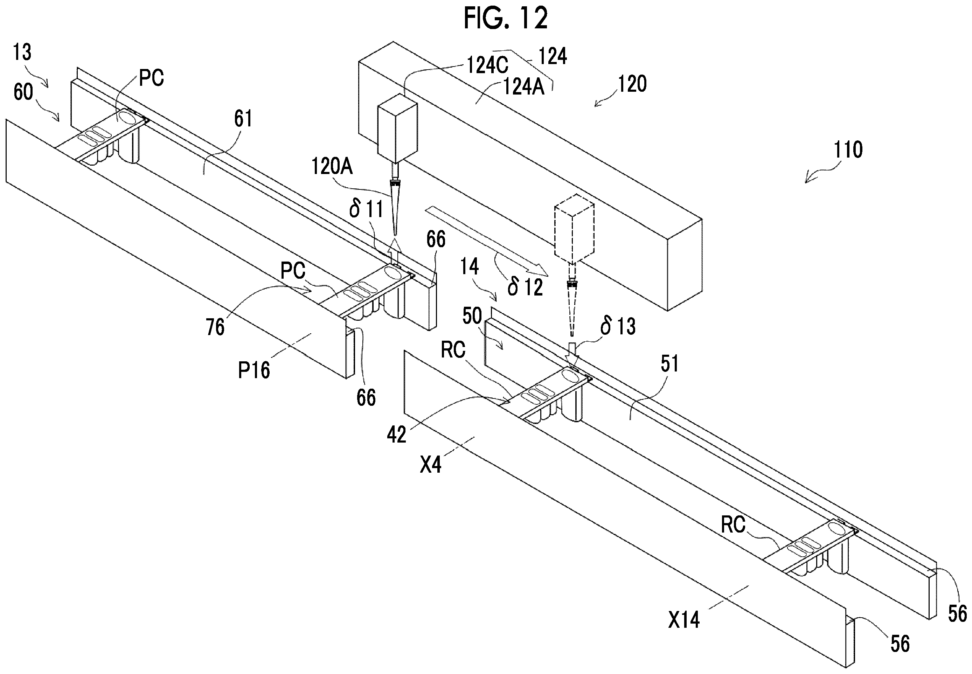

Fig. 12 is a perspective view showing a schematic configuration of a pre-treatment line 13, a measurement line 14, and a dispensing mechanism 120 in an examination apparatus 110 according to a second embodiment. In Fig. 12, the same constituent elements as those of the examination apparatus 10 are denoted by the same reference numerals, and detailed description thereof will be omitted. Hereinafter, the examination apparatus 110 according to the second embodiment will be described mainly with differences from the examination apparatus 10 according to the first embodiment.

In the examination apparatus 110, the transport passage 61 for pre-treatment of the pre-treatment line 13 and the transport passage 51 for measurement of the measurement line 14 are arranged in series so as to be connected in a straight line.

The dispensing mechanism 120 is an example of a specimen transfer mechanism same as the dispensing mechanism 12 in the examination apparatus 10. The dispensing mechanism 120 is a mechanism which dispenses a liquid, and includes a nozzle 120A which suctions and discharges the specimen 22, a nozzle moving mechanism 124, and a suction-discharge mechanism (not shown). The nozzle 120A is provided with a replaceable tip at a distal end thereof. The nozzle moving mechanism 124 includes an X direction movable part 124A, a Y direction movable part (not shown), and a Z direction movable part 124C, and is a mechanism that three-dimensionally moves the nozzle 120A in a vertical direction (Z direction) and a horizontal direction (X-Y direction). The suction-discharge mechanism is a mechanism that causes the nozzle 120A to perform a suction operation of suctioning the liquid and a discharge operation of discharging the liquid from the nozzle 120A. The nozzle moving mechanism 124 includes an actuator such as a motor, which generates a driving force, and a driving force transmission mechanism consisting of a gear, a drive belt, and the like. The suction-discharge mechanism is composed of a pump or the like, which generates a driving force for the suction and discharge.

The dispensing mechanism 120 suctions the pre-treated specimen 22 from the cartridge PC for pre-treatment transported to the pre-treatment end position 76 provided at the position P16 of the terminal end of the transport passage 61 for pre-treatment (see arrow δ11). Next, the dispensing mechanism 120 moves the nozzle 120A in the X direction as shown by the arrow δ12, and discharges the specimen 22 to the cartridge RC for measurement at the position X4 of the transport passage 51 for measurement (see arrow δ13).

In the examination apparatus 110, the pre-treatment end position 76 is fixed. Although not shown in Fig. 12, the examination apparatus 110 has a pre-treatment cartridge set mechanism 80 capable of setting the cartridge PC for pre-treatment at any position of the transport passage 61 for pre-treatment is provided as shown in Figs. 10 and 11. The pre-treatment cartridge set mechanism 80 can set the cartridge PC for pre-treatment at any position of the transport passage 61 for pre-treatment.

As described above, even in a case where the transport passage 61 for pre-treatment of the pre-treatment line 13 and the transport passage 51 for measurement of the measurement line 14 are arranged in series so as to be connected in a straight line, the same effects as those of the examination apparatus according to the first embodiment can be obtained. By including the pre-treatment line 13, the examination apparatus 110 can execute the pre-treatment. With the present configuration, the pre-treatment end position 76 in the transport passage 61 for pre-treatment and the specimen dispensing position 42 in the transport passage 51 for measurement can be arranged to be close to each other. Therefore, a transfer distance of the pre-treated specimen 22 can be set to be short, and occurrence of contamination due to the transfer of the pre-treated specimen 22 can be suppressed. In the examination apparatus 110, since the specimen transfer mechanism (here, the dispensing mechanism 120) that transfers the pre-treated specimen 22 from the cartridge PC for pre-treatment to the cartridge for measurement is provided, the pre-treatment can be performed for each examination item (examination target substance) in a suitable pre-treatment time.

In the examination apparatus 10 according to the first embodiment described above, the transport passage 61 for pre-treatment and the transport passage 51 for measurement are arranged such that the transport directions are parallel to each other and the downstream side of the transport passage 61 for pre-treatment and the upstream side of the transport passage 51 for measurement partially overlap each other. In addition, in the examination apparatus 110 according to the second embodiment, the transport passage 61 for pre-treatment of the pre-treatment line 13 and the transport passage 51 for measurement of the measurement line 14 are arranged in series so as to be connected in a straight line. The arrangement of the transport passage 61 for pre-treatment and the transport passage 51 for measurement is not limited thereto. It is sufficient that the transport passage 61 for pre-treatment and the transport passage 51 for measurement are arranged such that the pre-treatment end position 76 in the transport passage 61 for pre-treatment, at which the pre-treatment is completed, is closer to an upstream side in the transport direction of the transport passage 51 for measurement than a downstream side in the transport direction.

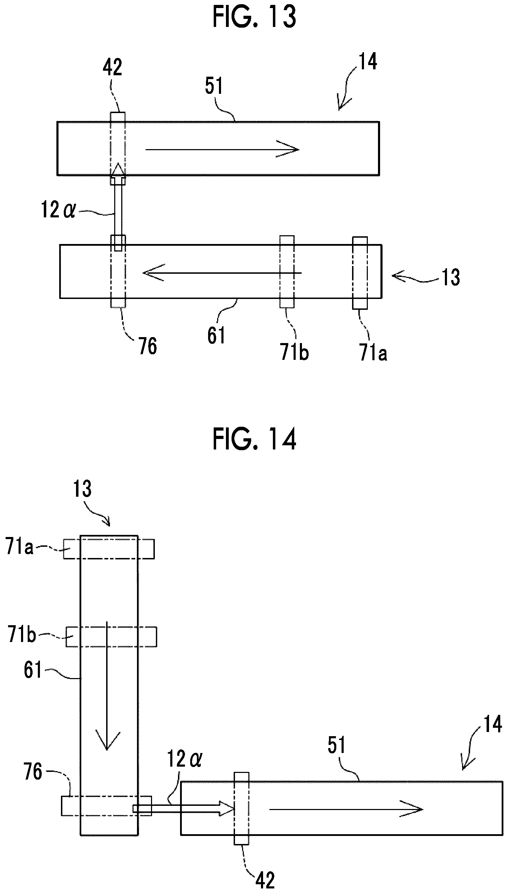

Modification examples of the arrangement configuration of the transport passage 61 for pre-treatment and the transport passage 51 for measurement will be described with reference to Figs. 13 to 19.

As shown in Fig. 13, the transport passage 61 for pre-treatment of the pre-treatment line 13 and the transport passage 51 for measurement of the measurement line 14 may be arranged such that the transport directions are parallel to each other and the upstream side and the downstream side of each transport direction are opposite to each other. With such an arrangement, the pre-treatment end position 76 of the transport passage 61 for pre-treatment and the specimen dispensing position 42 of the transport passage 51 for measurement can be arranged at the shortest distance. It is possible to suppress the contamination due to dripping or the like in a case where the pre-treated specimen 22 is transferred from the cartridge PC for pre-treatment at the pre-treatment end position 76 to the cartridge RC for measurement at the specimen dispensing position 42 (see arrow 12α). The arrow 12α indicates a movement path of the pre-treated specimen 22. The same applies to the following drawings. In the transport passage 61 for pre-treatment, the pre-treatment time can be adjusted by changing the cartridge set positions 71a and 71b, which are the pre-treatment start position. In addition, according to the present arrangement, even in a case where the transport passage 61 for pre-treatment is provided, a relatively small examination apparatus can be realized.

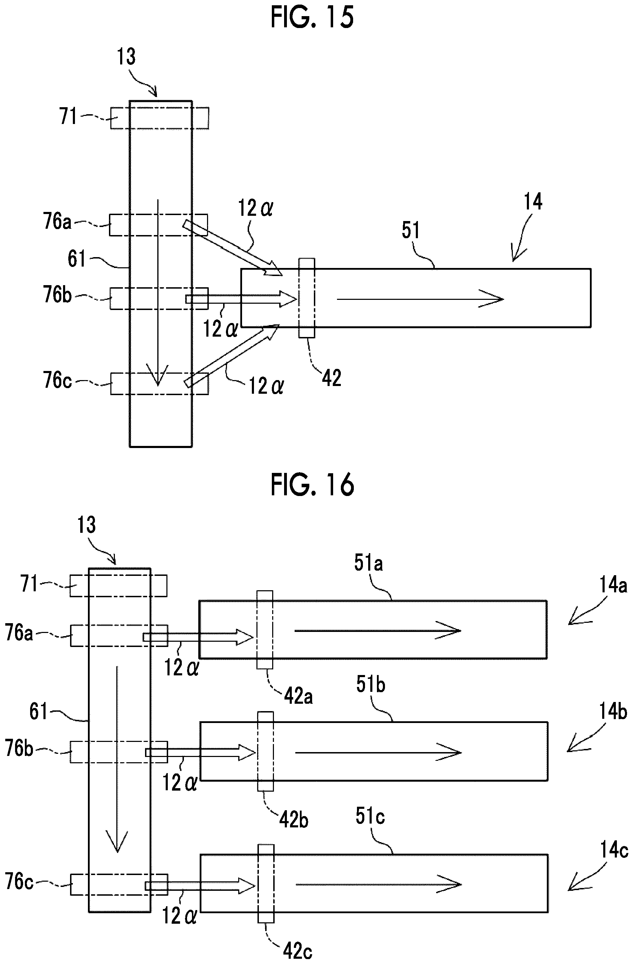

As shown in Figs. 14 and 15, the transport passage 61 for pre-treatment of the pre-treatment line 13 and the transport passage 51 for measurement of the measurement line 14 may be arranged such that the transport directions are orthogonal to each other.

In the modification example shown in Fig. 14, the transport passage 61 for pre-treatment and the transport passage 51 for measurement are arranged in an L-shape in which the terminal end of the transport passage 61 for pre-treatment and the start end of the transport passage 51 for measurement are close to each other. With such an arrangement, the pre-treatment end position 76 of the transport passage 61 for pre-treatment and the specimen dispensing position 42 of the transport passage 51 for measurement can be arranged to be close to each other. It is possible to suppress the contamination due to dripping or the like in a case where the pre-treated specimen 22 is transferred from the cartridge PC for pre-treatment at the pre-treatment end position 76 to the cartridge RC for measurement at the specimen dispensing position 42 (see arrow 12α). Even in the present example, in the transport passage 61 for pre-treatment, the pre-treatment time can be adjusted by changing the cartridge set positions 71a and 71b, which are the pre-treatment start position.

In the modification example shown in Fig. 15, the transport passage 61 for pre-treatment and the transport passage 51 for measurement are arranged in a substantially T-shape in which the start end of the transport passage 51 for measurement is close to the downstream side of the transport passage 61 for pre-treatment. In the present configuration, the cartridge set position 71 which is the pre-treatment start position is provided at the start end of the transport passage 61 for pre-treatment. The cartridge PC for pre-treatment is subjected to the pre-treatment according to the examination item, and is transported to pre-treatment end positions 76a, 76b, 76c, and the like according to the examination item. The pre-treated specimen 22 corresponding to the examination item is transferred from each of the pre-treatment end positions 76a, 76b, and 76c to the cartridge RC for measurement at the specimen dispensing position 42 of the transport passage 51 for measurement. According to the present configuration, a distance from the different pre-treatment end positions 76a, 76b, and 76c to the specimen dispensing position 42 can be made relatively short, and thus the contamination due to dripping or the like can be suppressed.

The transport directions of the transport passage 61 for pre-treatment and the transport passage 51 for measurement are not limited to being parallel or orthogonal to each other; and the transport passage 61 for pre-treatment and the transport passage 51 for measurement may be arranged such that the transport directions intersect each other.

In addition, as shown in Figs. 16 to 18, at least one of the transport passage 61 for pre-treatment of the pre-treatment line 13 or the transport passage 51 for measurement of the measurement line 14 may be provided in plurality.

In the modification example shown in Fig. 16, one pre-treatment line 13 includes a plurality (here, three) of measurement lines 14a, 14b, and 14c. Transport passages 51a, 51b, and 51c for measurement are arranged in a direction orthogonal to the transport passage 61 for pre-treatment. The three transport passages 51a, 51b, and 51c for measurement are arranged in parallel such that the respective start ends of the three transport passages 51a, 51b, and 51c for measurement are close to the side of the transport passage 61 for pre-treatment. In the present configuration, the cartridge set position 71 which is the pre-treatment start position is provided at the start end of the transport passage 61 for pre-treatment. The cartridge PC for pre-treatment is subjected to the pre-treatment according to the examination item, and is transported to pre-treatment end positions 76a, 76b, 76c, and the like according to the examination item. The pre-treated specimen 22 according to the examination item is transferred to specimen dispensing positions 42a, 42b, or 42c closest to the pre-treatment end position 76a, 76b, or 76c of the specimen dispensing positions 42a, 42b, and 42c of the three transport passages 51a, 51b, and 51c for measurement. According to the present configuration, a distance from the different pre-treatment end positions 76a, 76b, and 76c to the specimen dispensing position 42 can be made relatively short, and thus the contamination due to dripping or the like during the transfer of the pre-treated specimen 22 can be suppressed. In addition, it is possible to efficiently perform the processes on a plurality of specimens 22.

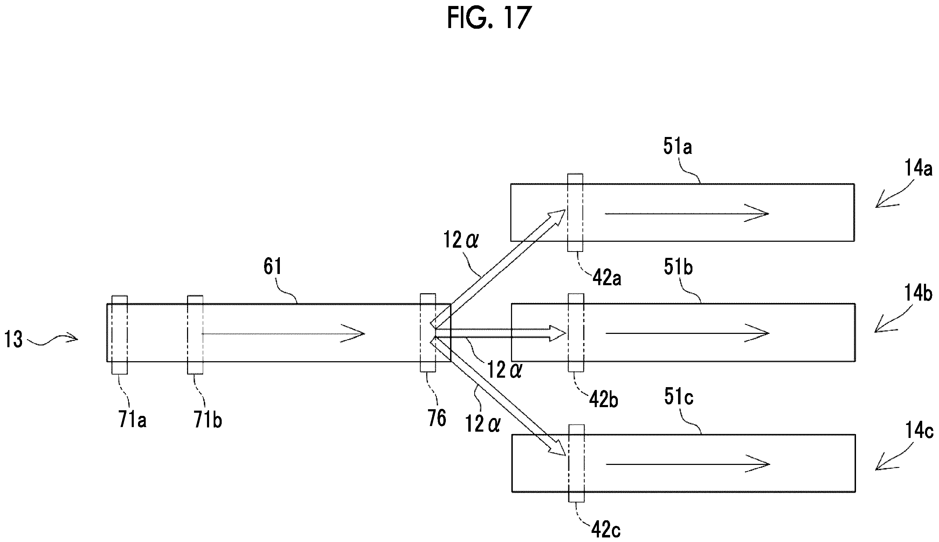

Even in the modification example shown in Fig. 17, one pre-treatment line 13 includes a plurality (here, three) of measurement lines 14a, 14b, and 14c. Transport passages 51a, 51b, and 51c for measurement are arranged in series with the transport passage 61 for pre-treatment. The three transport passages 51a, 51b, and 51c for measurement are arranged in parallel. The transport passage 61 for pre-treatment is disposed in series with the transport passage 51b for measurement disposed in the middle of the three transport passages 51a, 51b, and 51c for measurement arranged in parallel. Accordingly, the terminal end of the transport passage 61 for pre-treatment is disposed in a state of being close to the start end of any of the three transport passages 51a, 51b, and 51c for measurement. In the transport passage 61 for pre-treatment, the pre-treatment end position 76 is provided at the terminal end. Therefore, a distance from the pre-treatment end position 76 of the transport passage 61 for pre-treatment to the specimen dispensing position 42 of the transport passage 51 for measurement can be made relatively short. Therefore, it is possible to suppress the contamination due to the transfer of the treated specimen 22. In the transport passage 61 for pre-treatment, cartridge set positions 71a and 71b which are the pre-treatment start position can be set to any positions for each examination item. By providing the plurality of measurement lines 14a, 14b, and 14c, the examination efficiency can be improved.

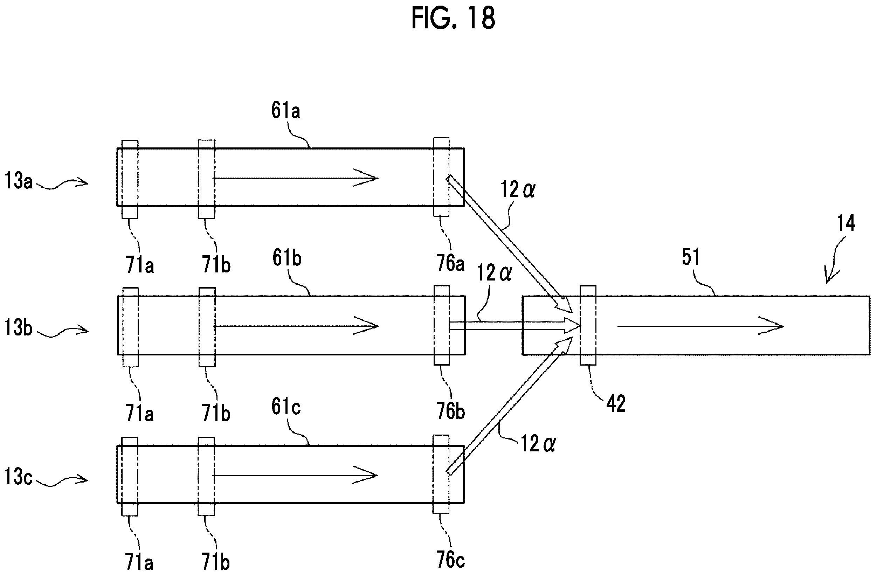

In the modification example shown in Fig. 18, one measurement line 14 is provided for three pre-treatment lines 13a, 13b, and 13c. Three transport passages 61a, 61b, and 61c for pre-treatment are arranged in parallel. The transport passage 51 for measurement is disposed in series with the transport passage 61b for pre-treatment disposed in the middle of the three transport passages 61a, 61b, and 61c for pre-treatment arranged in parallel. Accordingly, all terminal ends of the three transport passages 61a, 61b, and 61c for pre-treatment are arranged close to the start end of the transport passage 51 for measurement. In each of the transport passages 61a, 61b, and 61c for pre-treatment, pre-treatment end positions 76a, 76b, and 76c are provided at the terminal end. Therefore, a distance from each of the pre-treatment end positions 76a, 76b, and 76c of the transport passages 61a, 61b, and 61c for pre-treatment to the specimen dispensing position 42 of the transport passage 51 for measurement can be made relatively short. Therefore, it is possible to suppress the contamination due to the transfer of the treated specimen 22. In each of the transport passages 61a, 61b, and 61c for pre-treatment, cartridge set positions 71a and 71b which are the pre-treatment start position can be set to any positions for each examination item. Since the plurality of pre-treatment lines 13a, 13b, and 13c are provided, various pre-treatment conditions such as changing the pre-treatment temperature according to the examination item can be supported.

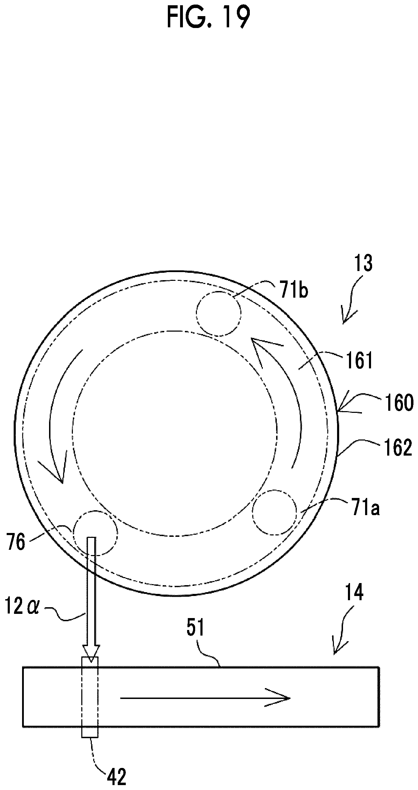

In each of the above-described examples, a case in which the transport passage 61 for pre-treatment is linear has been described. However, as shown in Fig. 19, the transport passage 61 for pre-treatment may have an annular shape. In the modification example shown in Fig. 19, a rotary transport mechanism 160 for pre-treatment is provided. The transport mechanism 160 for pre-treatment includes a rotary plate 162, a rotation mechanism (not shown), and a heating unit (not shown) such as a heat block disposed on the rotary plate 162. The cartridge for pre-treatment is placed on the rotary plate 162, and the rotary plate 162 is rotated so that the cartridge for pre-treatment is transported along a circumference of the rotary plate 162. An annular region along the circumference of the rotary plate 162 corresponds to a transport passage 161 for pre-treatment.

In the present example, the rotary plate 162 is disposed on a side of the measurement line 14 opposite to the transport passage 51 for measurement. In the transport passage 161 for pre-treatment, the pre-treatment end position 76 is set at a position close to the specimen dispensing position 42 on the transport passage 51 for measurement. In this way, in a case where the transport mechanism 160 for pre-treatment is a rotary type, the cartridge set positions 71a and 71b, which are the pre-treatment start position, are set at any position of the annular shape, so that the pre-treatment time can be appropriately set for each specimen 22. In addition, the cartridge for pre-treatment may be rotated once or more along the transport passage 161 for pre-treatment, and a degree of freedom of the pre-treatment time can be increased as compared with the linear transport passage.

In the above-described embodiments, the immunological analysis apparatus that detects the examination target substance included in the specimen by using the antigen-antibody reaction is described as an example. Needless to say, the technology of the present disclosure can be used for an examination apparatus other than the immunological analysis apparatus. In addition, the chemiluminescent immunoassay method has been described as an example of the method of detecting the examination target substance, but the present disclosure is not limited to this method and may be applied to other methods.

In addition, in the above-described embodiments, as a hardware structure of the processor, various processors shown below can be used. Various processors include a programmable logic device (PLD) that is capable of changing a circuit configuration after manufacturing, such as a field-programmable gate array (FPGA), and a dedicated electric circuit that is a processor having a circuit configuration dedicatedly designed for executing specific process, such as an application specific integrated circuit (ASIC), in addition to a CPU that is a general-purpose processor configured to execute software (program) to function as various processing units.

In addition, the above-described process may be executed by one of various processors or may be executed by a combination of two or more processors (for example, a combination of a plurality of FPGAs or a CPU and an FPGA) of the same type or different types. The plurality of processing units may be configured of one processor. As an example in which a plurality of processing units are composed of one processor, there is a form in which a processor that realizes all functions of a system including a plurality of processing units into one integrated circuit (IC) chip is used, such as system-on-chip (SOC).

Furthermore, the hardware structure of the processors is, more specifically, an electric circuit (circuitry) in which circuit elements such as semiconductor elements are combined.

In addition, the technology of the present disclosure is applied to not only the operation program of the examination apparatus but also a non-transitory computer readable storage medium (a USB memory, a digital versatile disc (DVD)-read only memory (ROM), or the like) storing the operation program of the examination apparatus.

The contents described and shown above are detailed descriptions of portions according to the technology of the present disclosure and are merely examples of the technology of the present disclosure. For example, the above description of the configurations, functions, operations, and effects is the description of examples of the configurations, functions, operations, and effects of portions related to the technology of the present disclosure. Therefore, unnecessary portions may be deleted or new elements may be added or replaced in the above descriptions and illustrations without departing from the gist of the technology of the present disclosure. In addition, in order to avoid complication and facilitate understanding of portions according to the technology of the present disclosure, description related to common technical knowledge or the like that does not need to be particularly described for enabling implementation of the technology of the present disclosure is omitted in the contents described and shown above.

The disclosure of Japanese Patent Application No. 2022-091058 filed on June 3, 2022 is incorporated in the present specification by reference. All cited documents, patent applications, and technical standards described in the specification are incorporated by reference in the specification to the same extent as in a case where each individual cited document, patent application, or technical standard is specifically and individually indicated to be incorporated by reference.

The following appendixes are further disclosed with respect to the above embodiments.

### (Appendix 1)

An examination apparatus for performing a measurement process using a cartridge for measurement, which has a reaction cell in which a specimen is dispensed and mixed with a reagent, the examination apparatus including:
a measurement line that has a transport passage for measurement, which linearly transports the cartridge for measurement in one direction from an upstream side to a downstream side, and that performs the measurement process while transporting the cartridge for measurement along the transport passage for measurement;
a pre-treatment line that performs a pre-treatment in which, before performing the measurement process, the specimen is heated in a state in which the specimen is mixed with a pre-treatment liquid, the pre-treatment line having a transport passage for pre-treatment, which transports a cartridge for pre-treatment used in the pre-treatment, the cartridge for pre-treatment being different from the cartridge for measurement, and the pre-treatment line performing the pre-treatment while transporting the cartridge for pre-treatment along the transport passage for pre-treatment; and
a specimen transfer mechanism that transfers a pre-treated specimen in which the pre-treatment is completed, from the cartridge for pre-treatment in the pre-treatment line to the cartridge for measurement in the measurement line,
in which the transport passage for pre-treatment and the transport passage for measurement are arranged such that a pre-treatment end position in the transport passage for pre-treatment, at which the pre-treatment is completed, is closer to the upstream side in a transport direction of the transport passage for measurement than the downstream side in the transport direction.

### (Appendix 2)

The examination apparatus according to the appendix 1,
in which the transport passage for pre-treatment is a transport passage that linearly transports the cartridge for pre-treatment in one direction from an upstream side to a downstream side, similarly to the transport passage for measurement.

### (Appendix 3)

The examination apparatus according to the appendix 2,
in which the transport passage for pre-treatment and the transport passage for measurement are arranged such that the downstream side in the transport direction of the transport passage for pre-treatment is closest to the upstream side in the transport direction of the transport passage for measurement.

### (Appendix 4)

The examination apparatus according to the appendix 2 or 3,
in which the transport passage for pre-treatment and the transport passage for measurement are arranged in series so as to be connected in a straight line.

### (Appendix 5)

The examination apparatus according to the appendix 2 or 3,
in which the transport passage for pre-treatment and the transport passage for measurement are arranged such that the transport directions are parallel to each other and the downstream side of the transport passage for pre-treatment and the upstream side of the transport passage for measurement partially overlap each other.

### (Appendix 6)

The examination apparatus according to the appendix 2 or 3,
in which the transport passage for pre-treatment and the transport passage for measurement are arranged such that the transport directions are parallel to each other and the upstream side and the downstream side of each transport direction are opposite to each other.

### (Appendix 7)

The examination apparatus according to the appendix 2 or 3,
in which the transport passage for pre-treatment and the transport passage for measurement are arranged such that the transport directions are orthogonal to each other.

### (Appendix 8)

The examination apparatus according to the appendix 2 or 3,
in which at least one of the transport passage for pre-treatment or the transport passage for measurement is provided in plurality.

### (Appendix 9)

The examination apparatus according to the appendix 1,
in which the transport passage for pre-treatment has an annular shape.

### (Appendix 10)

The examination apparatus according to any one of the appendixes 1 to 9,
in which, in the transport passage for pre-treatment, at least one of a pre-treatment start position at which the pre-treatment is started or the pre-treatment end position is variable.

### (Appendix 11)

The examination apparatus according to any one of the appendixes 1 to 10,
in which the specimen transfer mechanism is a dispensing mechanism that suctions the pre-treated specimen from the cartridge for pre-treatment and discharges the pre-treated specimen to the cartridge for measurement.

### (Appendix 12)

The examination apparatus according to the appendix 11,
in which the dispensing mechanism is capable of suctioning the pre-treated specimen from the cartridge for pre-treatment at any position on the transport passage for pre-treatment.

### (Appendix 13)

The examination apparatus according to any one of the appendixes 1 to 12,
in which the measurement line and the pre-treatment line each have a heating unit which heats the specimen, and
each heating unit is controllable to a different temperature.

## Claims

1. An examination apparatus for performing a measurement process using a cartridge for measurement, which has a reaction cell in which a specimen is dispensed and mixed with a reagent, the examination apparatus comprising:
a measurement line that has a transport passage for measurement, which linearly transports the cartridge for measurement in one direction from an upstream side to a downstream side, and that performs the measurement process while transporting the cartridge for measurement along the transport passage for measurement;
a pre-treatment line that performs a pre-treatment in which, before performing the measurement process, the specimen is heated in a state in which the specimen is mixed with a pre-treatment liquid, the pre-treatment line having a transport passage for pre-treatment, which transports a cartridge for pre-treatment used in the pre-treatment, the cartridge for pre-treatment being different from the cartridge for measurement, and the pre-treatment line performing the pre-treatment while transporting the cartridge for pre-treatment along the transport passage for pre-treatment; and
a specimen transfer mechanism that transfers a pre-treated specimen in which the pre-treatment is completed, from the cartridge for pre-treatment in the pre-treatment line to the cartridge for measurement in the measurement line,
wherein the transport passage for pre-treatment and the transport passage for measurement are arranged such that a pre-treatment end position in the transport passage for pre-treatment, at which the pre-treatment is completed, is closer to the upstream side in a transport direction of the transport passage for measurement than the downstream side in the transport direction.

2. The examination apparatus according to claim 1,
wherein the transport passage for pre-treatment is a transport passage that linearly transports the cartridge for pre-treatment in one direction from an upstream side to a downstream side, similarly to the transport passage for measurement.

3. The examination apparatus according to claim 2,
wherein the transport passage for pre-treatment and the transport passage for measurement are arranged such that the downstream side in the transport direction of the transport passage for pre-treatment is closest to the upstream side in the transport direction of the transport passage for measurement.

4. The examination apparatus according to claim 2,
wherein the transport passage for pre-treatment and the transport passage for measurement are arranged in series so as to be connected in a straight line.

5. The examination apparatus according to claim 2,
wherein the transport passage for pre-treatment and the transport passage for measurement are arranged such that the transport directions are parallel to each other and the downstream side of the transport passage for pre-treatment and the upstream side of the transport passage for measurement partially overlap each other.

6. The examination apparatus according to claim 2,
wherein the transport passage for pre-treatment and the transport passage for measurement are arranged such that the transport directions are parallel to each other and the upstream side and the downstream side of each transport direction are opposite to each other.

7. The examination apparatus according to claim 2,
wherein the transport passage for pre-treatment and the transport passage for measurement are arranged such that the transport directions are orthogonal to each other.

8. The examination apparatus according to claim 2,
wherein at least one of the transport passage for pre-treatment or the transport passage for measurement is provided in plurality.

9. The examination apparatus according to claim 1,
wherein the transport passage for pre-treatment has an annular shape.

10. The examination apparatus according to claim 1,
wherein, in the transport passage for pre-treatment, at least one of a pre-treatment start position at which the pre-treatment is started or the pre-treatment end position is variable.

11. The examination apparatus according to any one of claims 1 to 10,
wherein the specimen transfer mechanism is a dispensing mechanism that suctions the pre-treated specimen from the cartridge for pre-treatment and discharges the pre-treated specimen to the cartridge for measurement.

12. The examination apparatus according to claim 11,
wherein the dispensing mechanism is capable of suctioning the pre-treated specimen from the cartridge for pre-treatment at any position on the transport passage for pre-treatment.

13. The examination apparatus according to any one of claims 1 to 10,
wherein the measurement line and the pre-treatment line each have a heating unit which heats the specimen, and
each heating unit is controllable to a different temperature.
